(19) Europäisches Patentamt
European Patent Office
Office européen des brevets

(11) EP 4 230 637 A1

(12) EUROPEAN PATENT APPLICATION
published in accordance with Art. 153(4) EPC

(43) Date of publication:
**23.08.2023 Bulletin 2023/34**

(21) Application number: **21880216.3**

(22) Date of filing: **15.10.2021**

(51) International Patent Classification (IPC):
***C07K 1/13*** (2006.01) ***A61K 33/24*** (2019.01)
***A61K 33/244*** (2019.01) ***A61K 39/395*** (2006.01)
***A61K 41/00*** (2020.01) ***A61K 47/68*** (2017.01)
***A61K 47/69*** (2017.01) ***A61K 51/10*** (2006.01)
***A61P 35/00*** (2006.01) ***C07K 7/08*** (2006.01)
***C07K 16/28*** (2006.01) ***C07K 16/30*** (2006.01)
***C12N 15/11*** (2006.01)

(52) Cooperative Patent Classification (CPC):
**A61K 33/24; A61K 33/244; A61K 39/395; A61K 41/00; A61K 47/68; A61K 47/69; A61K 51/10; A61P 35/00; C07K 1/13; C07K 7/08; C07K 16/28; C07K 16/30; C12N 15/11**

(86) International application number:
**PCT/JP2021/038207**

(87) International publication number:
**WO 2022/080481 (21.04.2022 Gazette 2022/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.10.2020 JP 2020174840**
**24.12.2020 JP 2020215740**
**18.02.2021 JP 2021024688**

(71) Applicant: **Nihon Medi-Physics Co., Ltd**
**Koto-ku**
**Tokyo, 136-0075 (JP)**

(72) Inventors:
- **KAWATANI, Minoru**
**Tokyo 136-0075 (JP)**
- **HANADA, Takahisa**
**Tokyo 136-0075 (JP)**
- **TONOYA, Gota**
**Tokyo 136-0075 (JP)**
- **TAKEDA, Takuya**
**Tokyo 136-0075 (JP)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

# (54) RADIOACTIVE COMPLEXES OF ANTI-HER2 ANTIBODY, AND RADIOPHARMACEUTICAL

(57) The present invention provides a conjugate which has stability improved more than conventional conjugates without impairing the efficacy. The conjugate of the present invention is a conjugate of an anti-HER2 antibody site-specifically modified with a peptide and a chelating agent, wherein the chelating agent is chelated with a metal radionuclide, the peptide and the chelating agent are linked by a linker (L), and the linker (L) does not contain a thiourea bond.

EP 4 230 637 A1

## Description

[Technical Field]

The present invention relates to a radioconjugate of an anti-HER2 antibody, and a radiopharmaceutical.

[Background Art]

HER2 (Human Epidermal Growth Factor Receptor Type 2) is a growth factor receptor identified as a gene product of human oncogene HER2/neu, and is a transmembrane type protein with a molecular weight of about 185kDa.

As an anti-HER2 antibody, trastuzumab is known and is used clinically as an antitumor agent applicable to HER2 overexpressing breast cancer or gastric cancer.

It is known that trastuzumab is an antibody used in several ADCs (Antibody Drug Conjugates) on the market. ADC is a medicament in which a chemotherapeutic payload (drug) is covalently bonded to an antibody via a linker.

Antibody drugs have high target selectivity and relatively few side effects, but the efficacy thereof is sometimes insufficient. Chemotherapeutic agents have strong efficacy, but their low target selectivity increases the minimum effective dose necessary for killing cancer cells, and decreases the maximum tolerated dose because the dose cannot be increased much from the aspect of toxicity, thus causing a problem of narrow range of the therapeutic dosage.

According to ADC, higher amounts of chemotherapeutic agents can be selectively delivered to cancer cells. This is expected to result in wider therapeutic dosage ranges because lower doses achieve effects, chemotherapeutic agents that reach normal cells decrease, and the maximum tolerated doses increase.

One approach to ADC is a radioimmunoconjugate. In radioimmunoconjugates, radionuclides are used instead of payloads.

For example, Non Patent Literatures 1 and 2 describe $^{225}$Ac-labeled trastuzumab in which trastuzumab is randomly labeled with $^{225}$Ac by using 1,4,7,10-tetraazacyclododecane-1,4,7,10 tetraacetic acid (DOTA) as a chelating agent and reacting the isothiocyanate group introduced into DOTA with the terminal amino group of trastuzumab.

In addition, Patent Literature 1 describes $^{225}$Ac-labeled trastuzumab and $^{225}$Ac-labeled Pertuzumab, in which trastuzumab is randomly labeled with $^{225}$Ac, as anti-HER2 antibodies which are obtained by randomly modifying trastuzumab and pertuzumab with an azide group, and subjecting them to a click reaction with DOTAGA-DBCO labeled with Ac-225.

In addition, radioimmunoconjugates using radionuclides that emit gamma-ray and positron as radionuclides can be utilized for nuclear medicine examinations. Patent Literature 2 describes a conjugate of a peptide that site-specifically modifies the Fc region of an antibody and an anti-HER2 antibody. In addition, Patent Literature 3 describes that DTPA was introduced into this peptide to modify trastuzumab and trastuzumab was labeled with In-111 to obtain $^{111}$In-labeled trastuzumab, and that DFO was introduced into the peptide to modify trastuzumab and trastuzumab was labeled with Zr-89 to obtain $^{89}$Zr-labeled trastuzumab.

[Citation List]

[Patent Literature]

[PTL 1]
WO2019/125982
[PTL 2]
WO2016/186206
[PTL 3]
WO2017/217347

[Non Patent Literature]

[NPL 1]
Cancer Res.2003 Aug 15; 63(16):5084-90
[NPL 2]
Clin Cancer Res.2004 Jul 1; 10(13):4489-97

[Summary of Invention]

[Technical Problem]

However, it has been clarified from the findings of the present inventors that a radioconjugate of the anti-HER2 antibody, which forms a thiourea bond through the reaction between DOTA into which an isothiocyanate group has been introduced and an amino group, has problems such as low stability.

Patent Literature 1 does not describe site-specific modification of anti-HER2 antibody with peptides. In addition, it does not disclose or suggest the problem of anti-HER2 antibody that forms a thiourea bond.

[Solution to Problem]

One embodiment of the present invention is a conjugate of an anti-HER2 antibody site-specifically modified with a peptide and a chelating agent, wherein the chelating agent is chelated with a metal radionuclide, the peptide and the chelating agent are linked by a linker (L), and the linker (L) does not contain a thiourea bond.

Another embodiment of the present invention is a radiopharmaceutical containing the above-mentioned conjugate as an active ingredient.

In addition, another embodiment of the present invention is a radiopharmaceutical containing a conjugate of a chelating agent chelated with a metal radionuclide and an anti-HER2 antibody as active ingredients, wherein the linkage between the anti-HER2 antibody and the chelating agent does not contain a thiourea bond, and the conjugate has a radiochemical purity of not less than 90% when stored at room temperature for 7 days.

The "linkage" in the present invention means both direct connection and indirect connection, unless otherwise specified.

[Advantageous Effects of Invention]

According to the present invention, a radioconjugate of an anti-HER2 antibody is provided which has stability improved more than conventional conjugates without impairing the efficacy.

[Brief Description of Drawings]

[Fig. 1]
A graph showing changes in tumor volume over time in tumor-bearing mice of a radioconjugate (Example 1) group, a radioconjugate (Comparative Example 1) group, an antibody control group, and a vehicle group. The vertical axis indicates relative values when the tumor volume at the time of administration of each medicament is set to 1, and the horizontal axis indicates the number of days elapsed since administration of each medicament. The graph represents the mean±standard deviation of tumor volume in each group, "*" is the time point when a significant difference ($p<0.05$) was observed from the antibody control group; "**" is the time point when a significant difference ($p<0.01$) was observed from the antibody control group, "†" is the time point when a significant difference ($p<0.05$) was observed from the Vehicle group, and "‡" is the time point when a significant difference ($p<0.01$) was observed from the Vehicle group.

[Fig. 2]
A graph showing changes in body weight over time in tumor-bearing mice of a radioconjugate (Example 1) group, a radioconjugate (Comparative Example 1) group, an antibody control group, and a vehicle group. The vertical axis indicates relative values when the body weight at the time of administration of each medicament is set to 1, and the horizontal axis indicates the number of days elapsed since administration of each medicament. The graph represents the mean±standard deviation of body weight in each group.

[Fig. 3]
A graph showing evaluation results of the antigen-binding properties of radioconjugates produced according to Example 1 and Comparative Example 1. The vertical axis indicates values obtained by normalizing the value obtained by dividing the count value in the region of interest (ROI) set on the tumor section used by the area of the ROI, with the value obtained by dividing the count value of the standard radiation source by the area of the standard radiation source. The horizontal axis indicates the cell type of the tumor section used on each evaluation date. The graph represents the mean±standard deviation of each sample (n=10).

[Fig. 4]
A graph showing evaluation results of the antigen-binding properties of radioconjugates produced according to

Example 4 and Comparative Example 4. The vertical axis indicates values obtained by normalizing the value obtained by dividing the count value in the region of interest (ROI) set on the tumor section used by the area of the ROI, with the value obtained by dividing the count value of the standard radiation source by the area of the standard radiation source. The horizontal axis indicates the cell type of the tumor section used on each evaluation date. The graph represents the mean±standard deviation of each sample (n=10).

[Fig. 5]

A graph showing evaluation results of the antigen-binding properties of radioconjugates produced according to Example 5 and Comparative Example 5. The vertical axis indicates values obtained by normalizing the value obtained by dividing the count value in the region of interest (ROI) set on the tumor section used by the area of the ROI, with the value obtained by dividing the count value of the standard radiation source by the area of the standard radiation source. The horizontal axis indicates the cell type of the tumor section used on each evaluation date. The graph represents the mean±standard deviation of each sample (n=10).

[Fig. 6]

Images showing representative exemplary results of PET imaging of an SK-OV-3 cell subcutaneous tumor-bearing model mouse administered with a radioconjugate produced according to Example 2. Arrows in the Figure indicate the cancer-bearing tumor.

[Fig. 7]

A graph showing changes in tumor volume over time in tumor-bearing mice of each radioconjugate administration group and each ADC medicament administration group. The vertical axis indicates relative values when the tumor volume at the time of administration of each medicament is set to 1, and the horizontal axis indicates the number of days elapsed since administration of each medicament. The graph represents the mean±standard deviation of tumor volume in each group, "*" is the time point when a significant difference ($p<0.05$) was observed from an ENHERTU (registered trade mark) low dose group.

[Fig. 8]

A graph showing changes in tumor volume over time in tumor-bearing mice of each radioconjugate administration group, each ADC medicament administration group, an antibody control group, and a Vehicle group. The vertical axis indicates relative values when the tumor volume at the time of administration of each medicament is set to 1, and the horizontal axis indicates the number of days elapsed since administration of each medicament. The graph represents the mean±standard deviation of tumor volume in each group, "**" is the time point when a significant difference ($p<0.01$) was observed from the antibody control group, and "‡" is the time point when a significant difference ($p<0.01$) was observed from the Vehicle group.

[Description of Embodiments]

(1) Radioconjugate

The present invention is directed to a conjugate of an anti-HER2 antibody site-specifically modified with a peptide and a chelating agent, wherein the chelating agent is chelated with a metal radionuclide, the peptide and the chelating agent are linked by a linker (L), and the linker (L) does not contain a thiourea bond (hereinafter to be also referred to as the radioconjugate of the present invention).

(1-1) Metal radionuclide

The metal radionuclide contained in the radioconjugate of the present invention is a radionuclide that emits α-ray, a radionuclide that emits β-ray, a radionuclide that emits positron, or a radionuclide that emits γ-ray. When the radioconjugate of the present invention is used for cancer therapy, it is preferable to use a radionuclide that emits α-ray or a radionuclide that emits β-ray. When the radioconjugate of the present invention is used for cancer diagnosis or detection, it is preferable to use a radionuclide that emits positron, or a radionuclide that emits γ-ray. Examples of the radionuclide that emits α-ray include Bi-212, Bi-213, Ac-225, and Th-227. Examples of the radionuclide that emits β-ray include Cu-64, Y-90, and Lu-177. Examples of the radionuclide that emits positron include Cu-64, Ga-68, Y-86, and Zr-89. Examples of the radionuclide that emits γ-ray include Tc-99m and In-111. The metal radionuclide to be contained in the radioconjugate of the present invention is more preferably Ac-225, Y-90, Lu-177, or Zr-89.

(1-2) Antibody

The antibody to be contained in the radioconjugate of the present invention is an immunoglobulin that specifically binds to HER2 (hereinafter also to be referred to as the antibody used in the present invention). The antibody to be used in the present invention may be a polyclonal antibody or a monoclonal antibody, preferably a monoclonal antibody. The

origin of the antibody is not particularly limited, and examples include antibodies of non-human animals, non-human mammals, and humans, preferably human, rat, mouse, and rabbit. When the antibody is derived from species other than human, it is preferably chimerized or humanized using well-known techniques. The antibody to be used in the present invention may be a chimera antibody, a humanized antibody, or a human antibody. The antibody to be used in the present invention may be a bispecific antibody.

The antibody to be used in the radioconjugate of the present invention may be more preferably trastuzumab or pertuzumab.

Trastuzumab is a gene recombinant humanized monoclonal antibody in which the amino acid sequence of the antigen binding site of mouse monoclonal antibody (4D5) has been genetically replaced with the corresponding portion of human $IgG_1$. It consists of the complementarity determining region of mouse anti-human epithelial growth factor receptor type 2 (HER2) monoclonal antibody, the human framework region, and the constant region of human $IgG_1$. Trastuzumab is produced by Chinese hamster ovary cells produced.

In the present specification, trastuzumab is an antibody described in Japanese Translation of PCT Application Publication No. H6-508267, which is specifically a humanized antibody containing a light chain variable domain amino acid sequence:

DIQMTQSPSSLSASVGDRVTITCRASQDVNTAVAWY
QQKPGKAPKLLIYSASFLYSGVPSRFSGSRSGTDFT
LTISSLQPEDFATYYCQQHYTTPPTFGQGTKVEIKRT

(SEQ ID NO: 1)

and a heavy chain variable domain amino acid sequence:

EVQLVESGGGLVQPGGSLRLSCAASGFNIKDTYIHW
VRQAPGKGLEWVARIYPTNGYTRYADSVKGRFTISA
DTSKNTAYLQMNSLRAEDTAVYYCSRWGGDGFYAMD
YWGQGTLVTVSS ..

(SEQ ID NO: 2)

Trastuzumab is clinically used as an antitumor agent applicable to breast cancer with confirmed HER2 overexpression or unresectable advanced or recurrent gastric cancer with confirmed HER2 overexpression, and is available as Herceptin (registered trade mark), or various biosimilar products (Trastuzumab BS).

Herceptin (registered trade mark) and trastuzumab as a biosimilar product thereof are glycoproteins (molecular weight: about 148,000) consisting of 2 molecules of H chain (γ1 chain) consisting of 450 amino acid residues and 2 molecules of L chain (κ chain) consisting of 214 amino acid residues.

Pertuzumab is a gene recombinant humanized monoclonal antibody in which the amino acid sequence of the antigen binding site of mouse monoclonal antibody (2C4) has been genetically replaced with the corresponding portion of human $IgG_1$. It consists of the complementarity determining region of mouse anti-HER2 monoclonal antibody, the human framework region of human $IgG_1$, and the constant region of human $IgG_1$. Pertuzumab is produced by Chinese hamster ovary cells. Pertuzumab is clinically used as an antitumor agent applicable to HER2-positive breast cancer, and is available as PERJETA (registered trade mark). In PERJETA (registered trade mark), pertuzumab is a glycoprotein (molecular weight: about 148,000) consisting of 2 molecules of H chain (γ1 chain) consisting of 449 amino acid residues and 2 molecules of L chain (κ chain) consisting of 214 amino acid residues.

(1-3) Chelating agent

In the present invention, the chelating agent is not particularly limited as long as it has a site in the structure thereof where metal radionuclide is coordinated. Examples of the chelating agent include CB-TE2A (1,4,8,11-Tetraazabicyclo[6.6.2]hexadecane-4,11-diacetic acid), CDTA(Cyclohexane-trans-1,2-diamine tetra-acetic acid), CDTPA (4-cyano-4-[[(dodecylthio)thioxomethyl]thio]-Pentanoic acid), DOTA (1,4,7,10-Tetraazacyclododecane-1,4,7,10-tetraacetic acid), DOTMA ((1R,4R,7R,10R)-α,α',α'',α'''-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTA-GA (α-(2-Carboxyethyl)-1,4,7,10-tetraazacyclododecane-1,4,7,10-tetraacetic acid), DOTP (((1,4,7,10-Tetraazacyclododecane-1,4,7,10-tetrayl)tetrakis(methylene))tetraphosphonic acid), DOTMP (1,4,7,10-Tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid)),

D02P (Tetraazacyclododecane dimethanephosphonic acid), Deferoxamine (DFO), DTPA (Glycine, N,N-bis[2-[bis(carboxymethyl)amino]ethyl]-), CHX-A"-DTPA (2,2’-((2-(((1S,2R)-2-(bis(carboxymethyl)amino)cyclohexyl) (carboxymethyl)amino)ethyl) azanediyl)diacetic acid), DTPA-BMA (5,8-Bis(carboxymethyl)-11-[2-(methylamino)-2-oxoethyl]-3-oxo-2,5,8,11-tetraazatridecan-13-oic acid), EDTA (2,2’,2",2‴-(ethane-1,2-diylbis(azanetriyl))tetraacetic acid), NOTA (1,4,7-Triazacyclononane-1,4,7-triacetic acid), NOTP (1,4,7-Triazacyclononane-1,4,7-triyltris(methylenephosphonic acid)), TETPA (1,4,8,11-tetraazacyclotetradecane-1,4,8,11-tetrapropionic acid), TETA (1,4,8,11-Tetraazacyclotetradecane-N,N’,N",N‴-tetraacetic acid), TTHA (3,6,9,12-Tetrakis(carboxymethyl)-3,6,9,12-tetraazatetradecanedioic acid), HEHA (1,2,7,10,13-hexaazacyclooctadecane-1,4,7,10,13,16-hexaacetic acid), 1,2-HOPO (N,N’,N",N‴-tetra(1,2-dihydro-1-hydroxy-2-oxopyridine-6-carbonyl)-1,5,10,14-tetraazatetradecane), PEPA (1,4,7,10,13-pentaazacyclopentadecane-N,N’,N",N‴,N″″-penta-acetic acid), H4octapa (N,N’-bis(6-carboxy-2-pyridylmethyl)-ethylenediamine-N,N’-diacetic acid), H2bispa2 (6,6’-({9-hydroxy-1,5-bis(methoxycarbonyl)-2,4-di(pyridin-2-yl)-3,7-diazabicyclo[3.3.1]nonane-3,7-diyl} bis(-methylene))dipicolinic acid), H2dedpa (1,2-[{6-(carboxy)-pyridin-2-yl}-methylamino]ethane), H2macropa (6-(1,4,10,13-tetraoxa-7,16-diazacyclooctadecan-N,N’-methyl)picolinic acid), H5decapa (N,N"-bis(6-carboxy-2-pyridylmethyl)-diethylenetriamine-N,N’,N"-triacetic acid), H6phospa (N,N’-(methylenephosphonate)-N,N’-[6-(methoxycarbonyl)pyridin-2-yl]-methyl-1,2-diaminoethane), HP-D03A (Hydroxypropyltetraazacyclododecanetriacetic acid), and porphyrin. A compound represented by the following formula (A) is preferred.

(A)

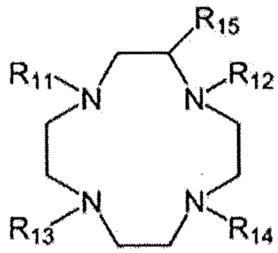

In the formula (A), $R_{11}$, $R_{12}$, $R_{13}$ and $R_{14}$ are each independently a group consisting of $-(CH_2)_pCOOH$, $-(CH_2)_pC_5H_5N$, $-(CH_2)_pPO_3H_2$, $-(CH_2)_pCONH_2$ or $-(CHCOOH)(CH_2)_pCOOH$, $R_{15}$ is a hydrogen atom, and p is an integer of not less than 0 and not more than 3.

The compound represented by the formula (A) is preferably a compound containing a structure derived from 1,4,7,10-tetraazacyclododecane-1,4,7,10-tetraacetic acid (DOTA), or a derivative thereof. Specifically, the compound can contain, in its structure, a structure derived from one chelating agent selected from DOTA (1,4,7,10-Tetraazacyclododecane-1,4,7,10-tetraacetic acid), DOTMA ((1R,4R,7R,10R)-α,α’,α",α‴-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTA-GA (α-(2-Carboxyethyl)-1,4,7,10-tetraazacyclododecane-1,4,7,10-tetraacetic acid), DOTP (((1,4,7,10-Tetraazacyclododecane-1,4,7,10-tetrayl)tetrakis(methylene))tetraphosphonicacid), DOTMP (1,4,7,10-Tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid)), and D02P (Tetraazacyclododecane dimethanephosphonic acid). More preferably, compounds represented by the following formulas (A-1) to (A-6) can be mentioned. The chelating agent to be used in the radioconjugate of the present invention is further preferably DOTA-GA (compound represented by the formula (A-6)).

(A-1)

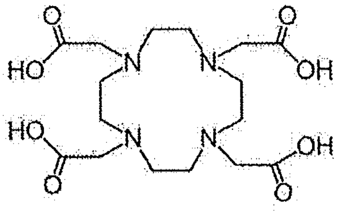

DOTA

(A-2)

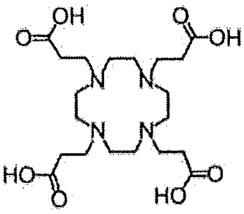

DOTPA

(A-3)

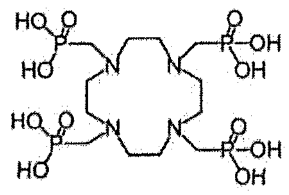

DOTMP

(A-4)

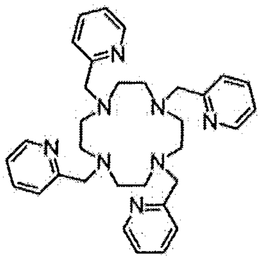

$L^{py}$

(A-5)

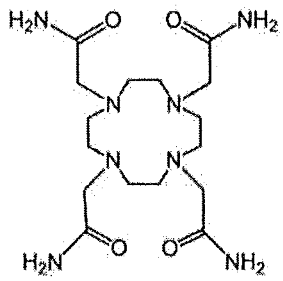

DOTAM

(A-6)

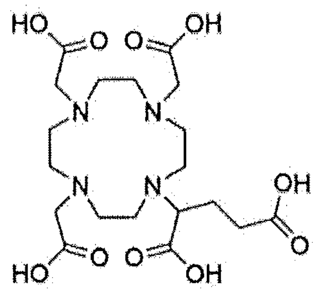

DOTA-GA

A chelating agent used in the present invention is linked with a peptide via a linker (L). In the radioconjugate of the present invention, the chelating agent and the linker (L) are preferably connected by a covalent bond. Therefore, in the radioconjugate of the present invention, some groups in the compound of the aforementioned chelating agent may be substituted by groups that form covalent bonds with the linker (L). For example, when the chelating agent used in the present invention is a compound represented by the formula (A), $R_{12}$ or $R_{15}$ may be substituted by a group that forms a covalent bond with the linker (L). Preferably, when $R_{12}$ is substituted by a group that forms a covalent bond with the linker (L), $R_{15}$ is a hydrogen atom, when $R_{12}$ is a group consisting of $-(CH_2)_pCOOH$, $-(CH_2)_pC_5H_5N$, $-(CH_2)_pPO_3H_2$, $-(CH_2)_pCONH_2$, or $-(CHCOOH)(CH_2)_pCOOH$, $R_{15}$ is substituted by a group that forms a covalent bond with the linker (L).

The covalent bond between the chelating agent and the linker (L) only needs to be free of a thiourea bond, and is exemplified by a carbon-carbon bond, an amide bond, an ether bond, an ester bond, and the like.

The connection between the chelating agent and the linker (L) can be formed, for example, by the reaction of an N-hydroxysuccinimido ester (NHS) group of the following formula (A-7) or (A-8), or a 2,6-dioxotetrahydro-2H-pyranyl group of the following formula (A-9), with the primary amine of linker (L) .

(A-7)

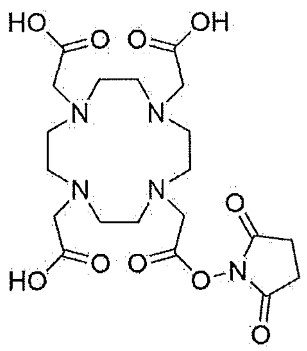

DO3A-NHS

(A-8)

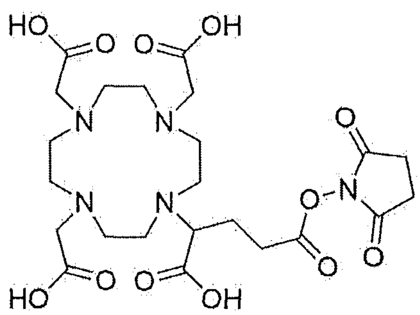

DOTA-GA-NHS

(A-9)

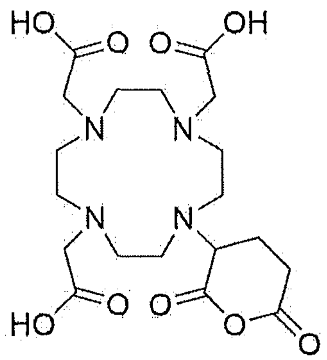

DOTA-GA-anhydride

(1-4) Antibody-modification peptide

In the present invention, the peptide is not particularly limited as long as it modifies the antibody site-specifically, preferably the Fc region site-specifically, more preferably the lysine residue in the Fc region of the antibody site-specifically. As a result, it is possible to maintain the activity of the antibody itself (antigen recognition action, neutralizing action, complement activating action and/or opsonin action).

The peptide to be used in the present invention may be a chain peptide or a cyclic peptide, and cyclic peptide is preferred. More preferably, it contains an amino acid sequence consisting of not less than 13 and not more than 17 amino acid residues represented by the following formula (i) (hereinafter, to be also referred to as "antibody-modification peptide"), and is modified with a crosslinking agent. In the explanation of the formula (i), the left side of the paper surface of the amino acid sequence indicates the N-terminal side, and the right side of the paper surface of the amino acid

sequence indicates the C-terminal side.

(Xa)-Xaa1-(Xb)-Xaa2-(Xc)-Xaa3-(Xd)··· (i)

In the formula (i), Xa, Xb, Xc and Xd are each continuous X in the number of a, continuous X in the number of b, continuous X in the number of c, and continuous X in the number of d, respectively,

X is an amino acid residue having neither a thiol group nor a haloacetyl group in the side chain,
a, b, c and d are each independently an integer of not less than one and not more than 5, and satisfy a+b+c+d≤14,
Xaa1 and Xaa3 are each independently an amino acid residue derived from an amino acid having a thiol group in the side chain, or an amino acid residue derived from an amino acid having a haloacetyl group in the side chain, wherein either Xaa1 or Xaa3 is an amino acid residue derived from an amino acid having a thiol group, preferably Xaa1 and Xaa3 are linked to form a ring structure, and
Xaa2 is a lysine residue, arginine residue, cysteine residue, aspartic acid residue, glutamic acid residue, 2-aminosuberic acid, or diamino propionic acid, preferably lysine residue, and Xaa2 is modified with a crosslinking agent.

Examples of the amino acid residue that may be contained in X in the above-mentioned formula (i) include those derived from amino acids such as glycine, alanine, phenylalanine, proline, asparagine, aspartic acid, glutamic acid, arginine, histidine, serine, threonine, tyrosine, methionine and the like, and X may be an amino acid residue consisting of the same type of amino acid, or different types of amino acids.
In the formula (i), a, b, c and d are not particularly limited as long as they are numbers within the aforementioned range. From the aspect of the stability of binding between the peptide and anti-HER2 antibody, a+b+c+d≤14 is to be satisfied, and a is preferably an integer of not less than 1 and not more than 3, b is preferably an integer of not less than 1 and not more than 3, c is preferably an integer of not less than 3 and not more than 5, and d is preferably an integer of not less than 1 and not more than 3.
At least one of Xaa1 and Xaa3 is an amino acid residue derived from an amino acid having a thiol group in the side chain, and Xaa1 and Xaa3 may be the same or different. Examples of the amino acid having a thiol group in the side chain include cysteine and homocysteine. Such amino acid residues are preferably bonded by a disulfide bond, or a sulfide group is preferably bonded thereto via a structure shown by the following formula (4). In the formula (4), the broken line indicates the binding part with the sulfide group.

(4) 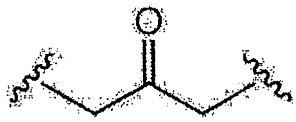

Instead of the aforementioned combination of Xaa1 and Xaa3, one of Xaa1 and Xaa3 may be an amino acid residue derived from an amino acid having a thiol group in the side chain, and the other may be an amino acid residue derived from an amino acid having a haloacetyl group in the side chain. These are bonded via a thioether bond. The terminal of the haloacetyl group is substituted with a halogen such as iodine or the like, and the halogen is eliminated by a reaction with the thiol group in the other side chain, whereby a thioether bond is formed.
Specific examples of the amino acid sequence of the antibody-modification peptide represented by the formula (i) include the peptides described in WO 2016/186206, WO 2017/217347 and WO 2018/230257, and these can also be used.
Preferably, the antibody-modification peptide to be used in the present invention is an amino acid sequence consisting of 13 to 17 amino acid residues represented by the following formula.

$(X_{1-3})$-C-(Xaa3')-(Xaa4')-H-(Xaa1')-G-(Xaa2')-L-V-W-C-$(X_{1-3})$

In the formula, each X is independently any amino acid residue other than cysteine,

C is a cysteine residue,
H is a histidine residue,
Xaa1' is a lysine residue, a cysteine residue, an aspartic acid residue, a glutamic acid residue, a 2-aminosuberic acid, or a diamino propionic acid,
G is a glycine residue,
Xaa2' is a glutamic acid residue or an asparagine residue,
L is a leucine residue,

V is a valine residue,
W is a tryptophan residue,
Xaa3' is an alanine residue, a serine residue, or a threonine residue, and
Xaa4' is a tyrosine residue or a tryptophan residue.

In the above-mentioned formula, N-terminal or C-terminal $X_{1-3}$ means that any 1 to 3 amino acid residues X other than cysteine (C or Cys) are independently consecutive, and is a sequence consisting of the same or different amino acid residues, preferably all three different amino acid residues.

Among these, the amino acid sequence of the antibody-modification peptide preferably has any one of the following sequences (1) to (14), more preferably the following sequence (1), (2), (13) or (14). In the following amino acid sequences (1) to (14), (Xaa2) is a lysine residue, a cysteine residue, an aspartic acid residue, a glutamic acid residue, a 2-aminosuberic acid, or a diamino propionic acid, preferably a lysine residue, (Xaa2) is preferably modified with a crosslinking agent, and (Xaa1) and (Xaa3) are each a homocysteine residue. In the following amino acid sequences (1) to (14), the amino acids other than (Xaa1), (Xaa2) and (Xaa3) are indicated by one-letter abbreviations.

(1) DCAYH(Xaa2)GELVWCT (SEQ ID NO: 3)
(2) GPDCAYH(Xaa2)GELVWCTFH (SEQ ID NO: 4)
(3) RCAYH(Xaa2)GELVWCS (SEQ ID NO: 5)
(4) GPRCAYH(Xaa2)GELVWCSFH (SEQ ID NO: 6)
(5) SPDCAYH(Xaa2)GELVWCTFH (SEQ ID NO: 7)
(6) GDDCAYH(Xaa2)GELVWCTFH (SEQ ID NO: 8)
(7) GPSCAYH(Xaa2)GELVWCTFH (SEQ ID NO: 9)
(8) GPDCAYH(Xaa2)GELVWCSFH (SEQ ID NO: 10)
(9) GPDCAYH(Xaa2)GELVWCTHH (SEQ ID NO: 11)
(10) GPDCAYH(Xaa2)GELVWCTFY (SEQ ID NO: 12)
(11) SPDCAYH(Xaa2)GELVWCTFY (SEQ ID NO: 13)
(12) SDDCAYH(Xaa2)GELVWCTFY (SEQ ID NO: 14)
(13) RGNCAYH(Xaa2)GQLVWCTYH (SEQ ID NO: 15)
(14) G(Xaa1)DCAYH(Xaa2)GELVWCT(Xaa3)H (SEQ ID NO: 16)

The peptide represented by the above-mentioned formula (i) or the peptide having the sequences (1) to (14) preferably has a linker (L) introduced at the N-terminal and is amidated at the C-terminal. In addition, Xaa2 of these peptides has been modified with a crosslinking agent, which allows for covalent bonding of the peptides to the Fc region of human IgG or rabbit IgG via the crosslinking agent. In the present specification, the "human anti-HER antibody" means an anti-HER antibody in which a region capable of binding an antibody-modification peptide is conserved in human IgG, preferably an anti-HER2 antibody in which Fc region of human IgG is conserved.

A crosslinking agent can be appropriately selected by those of ordinary skill in the art, and can be a compound having at least two sites bindable to desired amino acids (e.g., lysine, cysteine, aspartic acid, glutamic acid, 2-aminosuberic acid, diaminopropionic acid, arginine, etc.). Examples thereof include, but are not limited to, a crosslinking agent preferably containing two or more succinimidyl groups such as DSG (disuccinimidyl glutarate), DSS (disuccinimidyl suberate), and the like, a crosslinking agent preferably containing two or more imide acid moieties such as DMA (dimethyl adipimidate·2HCl, dimethyl adipimidate dihydrochloride), DMP (dimethyl pimelimidate·2HCl, dimethyl pimelimidate dihydrochloride), DMS (dimethyl suberimidate·2HCl, dimethyl suberimidate dihydrochloride), and the like, and a crosslinking agent having an SS bond such as DTBP (dimethyl 3,3'-dithiobispropionimidate·2HCl, dimethyl 3,3'-dithiobispropionimidate dihydrochloride) and DSP (dithiobis(succinimidyl propionate)), and the like, and SBAP (succinimidyl 3-(bromoacetamido)propionate). A crosslinking agent containing a succinimidyl group such as DSS or DSG reacts with a primary amine present at the N-terminal. Therefore, by blocking the N-terminal and reacting with DSS or DSG, only the amino group of Xaa2 can be specifically modified with DSS or DSG. For example, linker (L) may be previously introduced into the N-terminal of the antibody-modification peptide and then reacted with DSS or DSG. The succinimidyl group of DSS or DSG reacts with the Lys248 residue or Lys246 residue, preferably Lys248 residue, according to the Eu numbering in a human anti-HER2 antibody (e.g., trastuzumab), whereby the human anti-HER2 antibody is site-specifically modified with peptide. These Lys residues are present in the Fc region of human IgG, and even if the antibody is anti-HER2 antibody other than trastuzumab, those skilled in the art can align the amino acid sequence of the antibody and identify the corresponding Lys residue.

(1-5) Linker (L)

Linker (L) is not particularly limited as long as it can link a chelating agent and a peptide in the radioconjugate

of the present invention. Linker (L) to be used in the present invention is not particularly limited as long as it does not contain a thiourea bond. Examples thereof include substituted or unsubstituted alkyl group, substituted or unsubstituted heteroalkyl group, polyethylene glycol (PEG) group, peptides, sugar chain, disulfide group, amide group, combination of these and the like.

In the present specification, linker (L) is a general term for linkers used for the connection of an anti-HER2 antibody modified with a peptide and a chelating agent, and includes antibody-modification linker ($L_1$) and chelate linker ($L_2$). The antibody-modification linker ($L_1$), which is described in detail later, is introduced into the N-terminal side of the peptide described in (1-4), and the chelate linker ($L_2$), which is described in detail later, is introduced into the functional group of the chelating agent described in (1-3).

The linker (L) used in the present invention may contain a binding site formed by a click reaction, and preferably, the antibody-modification linker ($L_1$) and the chelate linker ($L_2$) are bound by a click reaction. In the present invention, it is preferred that a thiourea bond is not contained between the binding site formed by the click reaction and the chelating agent. In other words, it is preferred that the chelate linker ($L_2$) does not contain a thiourea bond. As used herein, the binding site formed by the click reaction is preferably a triazole skeleton-containing structure represented by the following formula (10a) or (10b) or a pyridazine skeleton-containing structure represented by the following formula (10c) can be considered. Since the formula (10a) and the formula (10b) are isomers, they may be contained at any ratio.

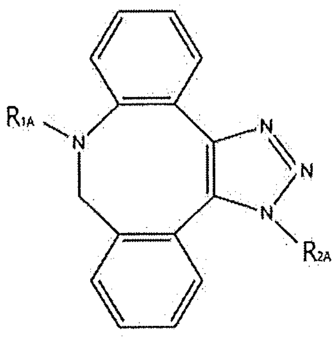

(10a)

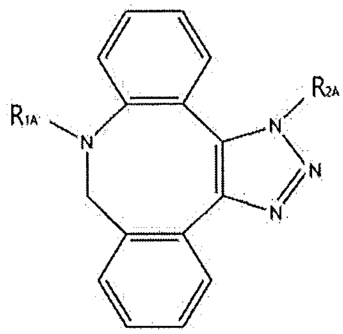

(10b)

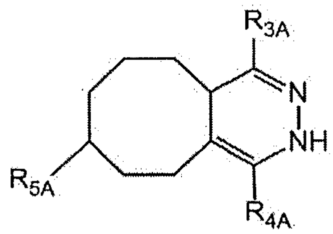

(10c)

In the formula (10a) and the formula (10b), $R_{1A}$ is a linkage site with a chelating agent, and $R_{2A}$ is a linkage site with an antibody-modification peptide. In the formula (10c), one of $R_{3A}$ and $R_{4A}$ is a hydrogen atom, a methyl group, a phenyl group or a pyridyl group, and the other is a linkage site with a chelating agent, and $R_{5A}$ is a linkage site with an antibody-modification peptide. In the formula (10a), the formula (10b), and the formula (10c), the linkage site with the antibody-modification peptide is linked with the peptide via the antibody-modification linker ($L_1$), and the linkage site with the chelating agent is linked with the chelating agent via the chelate linker ($L_2$)

In the radioconjugate of the present invention, the antibody is site-specifically modified with a peptide, and the peptide and a chelating agent are linked via a linker (L). Thus, one molecule or two molecules of the chelating agent are conjugated to one molecule of the anti-HER2 antibody.

(1-6) Production method of conjugate

The production method of the radioconjugate of the present invention includes two steps which are a conjugation step of conjugating a chelating agent and an anti-HER2 antibody, and a complex formation step of forming a complex of a metal radionuclide and a chelating agent. The conjugation step may be performed before the complex formation step or after the complex formation step.

In the conjugation step, the Fc region of an antibody is site-specifically modified with a chelating agent or linker (L) having the antibody-modification peptide shown in the aforementioned formula (i).

In the complex formation step, the chelating agent is chelated with a metal radionuclide (complex formation). The metal radionuclide used here is preferably used in a manner permitting ionization, more preferably in the form of an ion, from the viewpoint of increasing the complex formation efficiency. In the complex forming step, the order of addition of the metal radionuclide to the chelating agent does not matter as long as a complex can be formed with the metal radionuclide. For example, a solution in which radioactive metal ions are dissolved in a solvent mainly composed of water can be used as a radionuclide.

After complex formation, the obtained complex may be purified using a filtration filter, a membrane filter, a column filled with various fillers, chromatography or the like.

In the production method of the radioconjugate of the present invention, a conjugation step is preferably performed after the complex formation step.

In a more preferred embodiment, in complex formation step (A), a complex is formed between a metal radionuclide and a chelating agent having a first atomic group capable of click reaction as a substituent for enabling conjugate formation with the antibody. Then, in conjugation step (B), using an antibody-modification peptide shown by the aforementioned formula (i) and an antibody-modification linker ($L_1$) having a second atomic group capable of click reaction, a click reaction is performed between the peptide-modification antibody in which Fc region is site-specifically modified and the chelating agent with a formed complex which is obtained in step (A) to obtain the radioconjugate of the present invention.

The steps (A) and (B) are described in detail below.

As the combination of the first atomic group and the second atomic group capable of click reaction, an appropriate combination is selected according to the type of the click reaction. For example, a combination of alkyne and azide, a combination of 1,2,4,5-tetrazine and alkene, and the like can be mentioned. In these atomic groups, the first atomic group has one of the above-mentioned atomic group combination, and the second atomic group has one atomic group which is different from the first atomic group of the above-mentioned atomic group combination. To achieve both the stability of the chelating agent and the antibody and the improvement of the binding efficiency thereof, the chelate linker ($L_2$) is preferably alkyne and the antibody-modification linker ($L_1$) is preferably azide, or the chelate linker ($L_2$) is preferably 1,2,4,5-tetrazine and the antibody-modification linker ($L_1$) is preferably alkene. Specific examples of the click reaction by such combinations of atomic groups include a Husgen cyclization addition reaction, an inverse electron-requested Diels-Alder reaction, and the like.

Specific examples of the combination of the atomic groups capable of click reaction include, as shown in the following formulas, a combination of an atomic group containing dibenzylcyclooctyne (DBCO) as alkyne of the first atomic group (the formula (1a)) and an atomic group containing an azide group as azide of the second atomic group (the formula (2a)), and a combination of an atomic group containing 1,2,4,5-tetrazine as the first atomic group (the formula (1b)) and an atomic group containing trans-cyclooctene (TCO) as alkene of the second atomic group (the formula (2b)). Preferred is the combination of the formula (1a) and the formula (2a).

(1a) 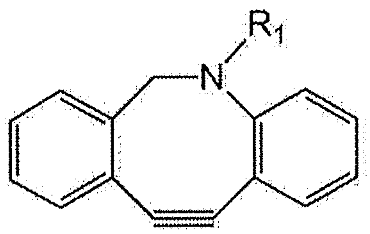

Dibenzylcyclooctyne

(2a) 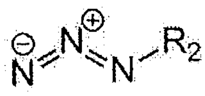

Azide

In the formula (1a), $R_1$ is a linkage site with a chelating agent, and in the formula (2a), $R_2$ is a linkage site with an antibody-modification peptide.

(1b) 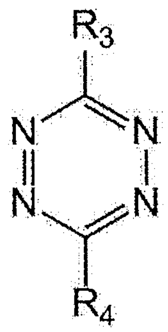

1,2,4,5-tetrazine

(2b) 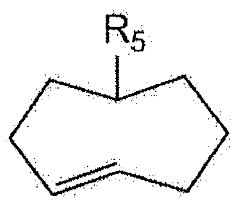

*trans*-cyclooctene

In the formula (1b), one of $R_3$ and $R_4$ is a linkage site with a chelating agent or an antibody-modification peptide, and the other is a hydrogen atom, a methyl group, a phenyl group or a pyridyl group. When the atomic group in the formula (1b) is linked to the chelating agent, $R_5$ is a linkage site to an antibody-modification peptide and when the atomic

group in the formula (1b) is linked to the antibody-modification peptide, $R_5$ is a linkage site to the chelating agent.

When an atomic group containing dibenzylcyclooctyne (DBCO) represented by the above-mentioned formula (1a) as alkyne of the first atomic group is used, various commercially available DBCO reagents can be mentioned. Specifically, for example, DBCO-C6-Acid, Dibenzylcyclooctyne-Amine, Dibenzylcyclooctyne Maleimide, DBCO-PEG acid, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, DBCO-mPEG and the like can be selected, and Dibenzylcyclooctyne Maleimide is preferably used.

In complex formation step (A), more preferably, a compound having a structure represented by the following formula (ii) is used.

A-B-C ··· (ii)

In the formula (ii), A is the aforementioned chelating agent, and the generic term of B and C is a chelate linker ($L_2$)

In the formula (ii), B is represented by the following formula (iib).

(iib) 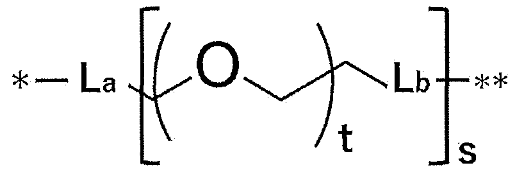

In the formula (iib), La and Lb are each independently a bond linker containing at least an amide bond and not less than 1 and not more than 50 carbon atoms, t is an integer of not less than 0 and not more than 30, s is 0 or 1, * is a binding site with A, and ** is a binding site with C.

In the formula (ii), C is either an alkyne derivative represented by the following formula (iic) or a tetrazine derivative represented by the formula (iid).

(iic) 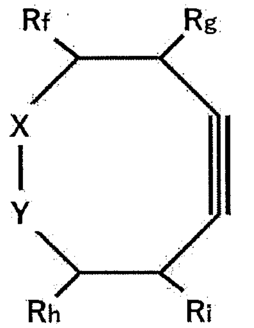

(iid) 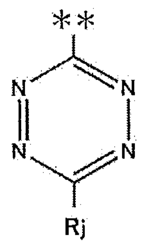

In the formula (iic), X is CHRk-** or N-**, Y is CHRk or C=O, Rk is independently a hydrogen atom or an alkyl group having not less than 1 and not more than 5 carbon atoms, when X is CHRk-** and Y is CHRk, then Rk moieties may be joined to form a cycloalkyl group, Rf, Rg, Rh and Ri are each independently a hydrogen atom, a halogen atom, or an alkyl group having not less than 1 and not more than 5 carbon atoms, Rf and Rg may be joined, or Rh and Ri may be joined to form a hydrocarbon ring, ** is a binding site with B, in the formula (iid), ** is a binding site with B, and Rj is a hydrogen atom, a methyl group, a phenyl group or a pyridyl group.

As the chelating agent used in step (A), a DOTA derivative of the above-mentioned formula (A) wherein $R_{11}$ to $R_{14}$ are $-(CH_2)_pCOOH$, p is 1, $R_{15}$ is a binding site with B; or DO3A derivative or DOTAGA derivative wherein $R_{11}$ to $R_{14}$ are - $(CH_2)_pCOOH$, p is 1, $R_{12}$ is a binding site (*) with B, and $R_{15}$ is a hydrogen atom is more preferred.

In the formula (ii), a DO3A-PEGt-DBCO wherein A is the above-mentioned DO3A, in B, La is a bond linker containing an amide bond and having not less than 1 and not more than 50 carbon atoms, s is 0 or 1, when s is 1, t is an integer of not less than 0 and not more than 30, Lb is a bond linker containing an amide bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is preferred.

In the formula (ii), a DOTAGA-PEGt-DBCO derivative wherein A is the above-mentioned DOTAGA derivative, in B, La is a bond linker containing an amide bond and having not less than 1 and not more than 50 carbon atoms, s is 0 or 1, when s is 1, t is an integer of not less than 0 and not more than 30, Lb is a bond linker containing an amide bond

and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is preferred. More preferred is the following DOTAGA-DBCO.

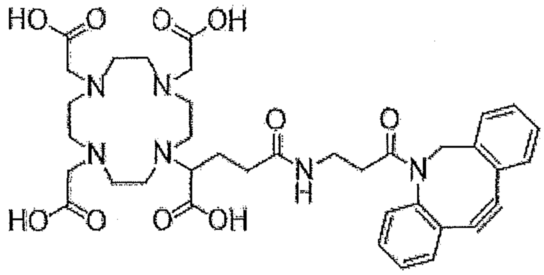

DOTAGA-DBCO

In the molar ratio of the chelating agent and metal radionuclide as chelate site/metal radionuclide, the lower limit is preferably not less than 10/1, more preferably not less than 100/1, further preferably not less than 500/1, and the upper limit is preferably not more than 10000/1, more preferably not more than 8000/1, further preferably not more than 7000/1. For example, the range of not less than 100/1 and not more than 7000/1 is preferred, and not less than 500/1 and not more than 7000/1 is more preferred.

The complex formation reaction is preferably performed in a solvent. As the solvent, water, saline, buffers such as sodium acetate buffer, ammonium acetate buffer, phosphate buffer, phosphate buffered saline, tris hydroxymethylaminomethane buffer (Tris buffer), 4-(2-hydroxyethyl)-1-piperazineethanesulfonic acid buffer (HEPES buffer), tetramethylammonium acetate buffer and the like, and the like can be used.

While the amount of the solvent is not particularly limited, from the aspect of practicality in the production step, the lower limit at the start of step (A) is not less than 0.01 mL, preferably not less than 0.1 mL, more preferably not less than 1.0 mL, further preferably not less than 10 mL, further more preferably not less than 100 mL, and upper limit is preferably not more than 1000 mL, more preferably not more than 100 mL, further preferably not more than 10 mL, further more preferably not more than 1.0 mL. For example, it is within the range of not less than 0.01 mL and not more than 100 mL.

As the concentration of the chelating agent in the reaction mixture of the complex formation reaction, from the aspect of the yield of the desired chelating agent, the lower limit at the start of step (A) is each independently preferably not less than 0.001 μmol/L, more preferably not less than 0.01 μmol/L, further preferably not less than 0.1 μmol/L, more preferably not less than 1 μmol/L, and the upper limit is preferably not more than 1000 μmol/L, more preferably not more than 100 μmol/L, further preferably not more than 10 μmol/L. For example, it is within the range of not less than 1 μmol/L and not more than 100 μmol/L.

The temperature of the complex formation reaction may be, for example, room temperature (25°C) or under heating conditions. To simultaneously achieve suppression of decomposition of the chelating agent and improvement of complex formation efficiency, the lower limit is preferably not less than 20°C, more preferably not less than 30°C, further preferably not less than 35°C, further more preferably not less than 37°C, particularly preferably not less than 45°C. The upper limit is preferably not more than 150°C, more preferably not more than 120°C, further preferably not more than 100°C, further more preferably not more than 90°C. For example, a range of not less than 30°C and not more than 100°C is preferred, and a range of not less than 35°C and not more than 90°C is more preferred.

The antibody to be used in step (B) is a peptide-modified antibody in which Fc region (constant region) of anti-HER2 antibody as described in detail in the above-mentioned "(1-2) Antibody" is site-specifically modified using the antibody-modification peptide shown in the aforementioned formula (i), and an antibody-modification linker ($L_2$) having the second atomic group capable of click reaction.

The antibody-modification peptide can be produced using a combination of amino acids regardless of natural amino acids and unnatural amino acids, by subjecting to peptide synthesis methods such as liquid phase synthesis process, solid phase synthesis process, automatic peptide synthesis method, gene recombinant method, phage display method and the like. In the synthesis of the peptide, where necessary, the functional groups of the amino acids to be used may be protected. These methods can be performed according to the method described in, for example, WO 2017/217347 and WO 2018/230257.

The antibody-modification linker ($L_2$) may be one in which an antibody-modification peptide and a linker ($L_2$) represented by the following formula (S1) are bonded.

$$*\text{-}((L_i)_m\text{-}Z)_k\text{-}L_{ii}\text{-}AG2 \cdots \quad (S1)$$

wherein * is a binding site with the N-terminal or C-terminal of peptide,

$L_i$ is a linker moiety of polyethylene glycol (PEG),
m is an integer of not less than 1 and not more than 50,
Z is a second linker moiety that binds $(L_i)_m$ and $L_{ii}$,
k is 0 or 1,
$L_{ii}$ is the second PEG linker moiety, and
AG2 is a second atomic group.

In the aforementioned formula (S1), the structure of Z is not particularly limited as long as it is a linker structure that binds $(L_i)_m$ and $L_{ii}$ to each other, and includes, for example, an amino acid sequence consisting of not less than 1 and not more than 5 amino acid residues. In this case, the amino acid sequence contained in Z preferably contains a cysteine residue, and is more preferably bonded to L2 via a thioether group formed by the bond between the thiol group of the cysteine residue and a maleimide group.

In the present invention, the polyethylene glycol (PEG) linker moiety constituting $L_{ii}$ preferably has the structure shown by the following formula (P2). In the formula (P2), n is an integer of preferably not less than 1 and not more than 50, more preferably not less than 1 and not more than 20, further preferably not less than 2 and not more than 10, further more preferably not less than 2 and not more than 6.

(P2) 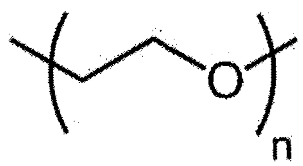

One end of the structure of the PEG linker moiety may be modified by a structure derived from a commercially available PEGylation reagent or a structure derived from a reagent generally used for PEGylation. Although not particularly limited, examples thereof include structures derived from diglycolic acid or a derivative thereof, and maleimide or a derivative thereof.

As a method for introducing the aforementioned second atomic group into an antibody-modification linker ($L_2$), an introduction method including obtaining an antibody-modification peptide having a desired amino acid sequence by the aforementioned method, dissolving the peptide in a solution containing a solubilizing agent and a reducing agent and, where necessary, an acid, adding an organic solvent solution of an atomic group containing an azide group or trans-cyclooctene (TCO) as the second atomic group to the solution, and stirring the mixture at room temperature can be mentioned.

When an atomic group containing an azide group is introduced as the second atomic group, the azide group is directly introduced into the N-terminal or C-terminal of a peptide by using a commercially available azide group-introducing reagent according to a conventional method, or an atomic group containing an azide group can be introduced via the aforementioned linker structure. Examples of the azide group-introducing reagent to be used include silyl azide, azide phosphate, alkyl ammonium azide, inorganic azide, sulfonyl azide, PEG azide, and the like.

When an atomic group containing TCO is introduced as the second atomic group, TCO is directly introduced into the N-terminal or C-terminal of a peptide by using a commercially available click chemistry reagent containing TCO according to a conventional method, or an atomic group containing TCO can be introduced via the aforementioned linker structure.

The method for binding an antibody-modification peptide to a human anti-HER2 antibody to obtain a peptide-modified antibody can be performed, for example, by dispersing the aforementioned antibody-modification peptide, a human anti-HER2 antibody, a crosslinking agent, and a catalyst as necessary in an appropriate buffer, according to the description of WO 2016/186206. As the crosslinking agent, those mentioned above can be used.

In one embodiment, the present disclosure relates to a method for producing a conjugate of an antibody-modification peptide and a human anti-HER2 antibody, comprising a step of mixing an antibody-modification peptide modified with a crosslinking agent and a human anti-HER2 antibody. By this step, a crosslinking reaction can occur between the antibody-modification peptide modified with the crosslinking agent and the human anti-HER2 antibody. The crosslinking reaction can occur site-specifically between the amino acid residue of the above-mentioned Xaa2 of the antibody-modification peptide and Lys248 or Lys246, preferably Lys248, according to Eu numbering in human IgG Fc.

The conditions of the mixing step are not particularly limited as long as a crosslinking reaction occurs between

the antibody-modification peptide and the human anti-HER2 antibody. For example, the reaction can be performed by mixing an antibody-modification peptide and a human anti-HER2 antibody in an appropriate buffer at room temperature (e.g., about 15°C to 30°C) . The mixing step may be performed by adding as necessary an appropriate amount of a catalyst that promotes the crosslinking reaction.

In one embodiment, a solvent containing at least water is added to dissolve the human anti-HER2 antibody. The solvent other than water includes, for example, dimethyl sulfoxide, acetonitrile, saline, and buffers such as sodium acetate buffer, ammonium acetate buffer, phosphate buffer, phosphate buffered saline, Tris buffer, HEPES buffer, tetramethylammonium acetate buffer, histidine buffer, and the like. When a buffer is used, the pH at 25°C is preferably set to 4.0 or more and 10.0 or less, more preferably 5.5 or more and 8.5 or less, from the aspect of the stability of the antibody. At the start of the crosslinking reaction, the concentration of the antibody is preferably set to 1.0 μmol/L or more as the lower limit and 1000 μmol/L or less, more preferably 500 μmol/L or less, as the upper limit.

Then, an antibody-modification peptide modified with a crosslinking agent and, where necessary, a catalyst are added and the mixture is dispersed at 10°C or higher and 30°C or lower.

The mixing ratio of the antibody-modification peptide and the human anti-HER2 antibody in the mixing step is not particularly limited. The molar ratio of the antibody-modification peptide to the human anti-HER2 antibody can be set to, for example, 1:1 to 20:1, preferably 2:1 to 20:1 or 5:1 to 10:1.

In a preferred embodiment, the molar ratio of the antibody-modification peptide to the human anti-HER2 antibody in the above-mentioned mixing step can be 0.5 to 2.2, preferably 0.8 to 1.8. In this way, an antibody in which one molecule of antibody-modification peptide is bound to one molecule of human anti-HER2 antibody (hereinafter referred to as "monovalent antibody") can be obtained efficiently.

The mixing time (reaction time) in the mixing step is not particularly limited as long as a crosslinking reaction occurs between the antibody-modification peptide and the human anti-HER2 antibody. It is, for example, 1 min to 5 hr, preferably 10 min to 2 hr.

The peptide-modification antibody obtained through the above steps is a mixture containing an antibody in which one molecule of antibody-modification peptide is bound to one molecule of human anti-HER2 antibody (i.e., monovalent antibody) and an antibody in which two molecules of antibody-modification peptide are bound to one molecule of human anti-HER2 antibody (hereinafter referred to as "divalent antibody") at any ratio. This may be used as it is for the subsequent steps, or an unmodified antibody, a monovalent antibody, and a divalent antibody are separated and purified by a method such as filtration filter, membrane filter, column filled with various fillers, various chromatographies and the like, and only the antibody having any valence may be subjected to the subsequent steps. When the unmodified antibody cannot be separated from the antibody having other valence as a result of purification, a mixture containing these may be subjected to the subsequent steps.

When an unmodified antibody, a monovalent antibody, and a divalent antibody are separated and purified, any of the above-mentioned purification methods may be used for separation and purification. It is possible to use a column packed with various fillers. For example, a column packed with a filler in which a protein such as protein A, protein G, or the aforementioned antibody-modification peptide is bound to a carrier can be used. The shape of the carrier of the filler packed in such a column includes gel (e.g., column gel), particle, bead, nanoparticle, microparticle, macrobead, and the like. The materials of the carrier include magnetic substance, latex, agarose, glass, cellulose, sepharose, nitrocellulose, polystyrene, and other polymeric materials. Specific example is an IgG-BP column in which the aforementioned antibody-modification peptide is bound to a column gel (see WO 2021/080008).

An IgG-BP column is a column in which an IgG-binding peptide is immobilized. Divalent antibody cannot bind to the column because the binding sites are already occupied by IgG-binding peptides, and only monovalent antibodies show affinity for the column. Using the IgG-BP column and utilizing the difference in the interaction with respective antibody-modification peptides, the first antibody composition containing relatively large amounts of the unmodified antibody and the monovalent antibody and the second antibody composition containing a relatively large amount of the divalent antibody can be respectively separated and purified. In one preferred embodiment, the molar ratio of unmodified antibody to monovalent antibody in the first antibody composition is 4-47:53-96, preferably 4-30:70-96, more preferably 4-20:80-96, further preferably 4-10:90-96.

The first antibody composition or the second antibody composition separated and purified in this manner may be used as it is for the click reaction in the subsequent step (B), or may be used for the click reaction in step (B) after adjusting the protein concentration of the peptide-modified antibody contained.

The click reaction in step (B) is performed between the first atomic group capable of click reaction which is contained in the chelating agent, and the second atomic group capable of click reaction which is contained in the peptide-modified antibody. By such click reaction, a binding group (substituent capable of conjugating with antibody) that links a chelating agent and an antibody is formed.

When the peptide-modified antibody and the complex obtained in step (A) are capable of click reaction, the order of addition of these does not matter. For example, one of the complex and the peptide-modified antibody is added to a reaction container containing a solvent, and then the other is added to perform the reaction, or one of the chelating

agent and the antibody is dispersed in a solvent and the other is added to the dispersion to perform the reaction. Alternatively, these may be simultaneously added to a reaction container containing a solvent to perform the reaction.

As the solvent to be used for the click reaction in step (B), a solvent containing water can be used. For example, water, saline, buffers such as sodium acetate buffer, ammonium acetate buffer, phosphate buffer, phosphate buffered saline, Tris buffer, HEPES buffer, tetramethylammonium acetate buffer and the like, and the like can be used. When a buffer is used, to simultaneously achieve the stability of the complex and the antibody, and the bond efficiency of these, the pH at 25°C is preferably set to not less than 4.0 and not more than 10.0, further preferably not less than 5.5 and not more than 8.5.

While the amount of the reaction mixture is not particularly limited, from the aspect of practicality in the production step, the lower limit at the start of step (B) is preferably not less than 0.001 mL, more preferably not less than 0.01 mL, further preferably not less than 0.1 mL, further more preferably not less than 1 mL, and the upper limit is preferably not more than 1000 mL, more preferably not more than 100 mL, further preferably not more than 10 mL, further more preferably not more than 1 mL. For example, the range of not less than 0.001 mL and not more than 1000 mL is preferable, and the range of not less than 0.1 mL and not more than 10 mL is more preferable.

As the concentrations of the chelating agent and the antibody in the reaction mixture, each independently, the lower limit at the start of step (B) is preferably not less than 0.001 μmol/L, more preferably not less than 0.01 μmol/L, further preferably not less than 0.1 μmol/L, further more preferably not less than 1.0 μmol/L, and the upper limit is preferably not more than 1000 μmol/L, more preferably not more than 100 μmol/L. For example, the range of not less than 0.1 μmol/L and not more than 1000 μmol/L is preferable, and the range of not less than 1 μmol/L and not more than 100 μmol/L is more preferable, from the aspect of the yield of the desired conjugate.

To prevent unintended denaturation of the antibody and increase the reaction efficiency, the upper limit of the reaction temperature of the click reaction in step (B) is preferably not more than 50°C, more preferably not more than 40°C. The lower limit of the reaction temperature is not particularly limited as long as the reaction proceeds, and is preferably not less than 15°C. The reaction time of the click reaction is, on the condition that it is the aforementioned reaction temperature, preferably not less than 5 min, more preferably not less than 10 min, preferably not more than 24 hr, more preferably not more than 20 hr. For example, the range of not less than 5 min and not more than 24 hr is preferable, and the range of not less than 10 min and not more than 20 hr is more preferable.

The obtained conjugate may be used as it is or purified using a filtration filter, a membrane filter, a column filled with various fillers, chromatography or the like.

In the conjugate produced by steps (A) and (B), the lysine residue in the Fc region of anti-HER2 antibody is specifically modified with a chelating agent. This conjugate comprises one or two molecules of the aforementioned chelating agent per one molecule of the antibody. The chelating agent site-specifically modifies the Fc region of the antibody of the present invention via a linker (L). The linker (L) is constituted of a chelate linker ($L_2$) that connects to a chelating agent, a first atomic group that connects to the linker ($L_2$), a second atomic group that can perform click reaction with the first atomic group, and an antibody-modification linker ($L_1$) that connects to the second atomic group (including an antibody-modification peptide represented by the above-mentioned formula (i)). Therefore, the linker (L) has a chemical structure derived from the first atomic group and the second atomic group. As such chemical structure, a triazole skeleton-containing structure represented by the aforementioned formula (10a) or (10b) or a pyridazine skeleton-containing structure represented by the following formula (10c) can be considered. Since the formula (10a) and the formula (10b) are isomers, they may be contained at any ratio.

(1-7) Radiopharmaceutical (Radiopharmaceutical (1))

A radiopharmaceutical refers to a composition that contains the radioconjugate of the present invention and is in a form suitable for in vivo administration to a subject. The radiopharmaceutical may be, for example, a radioconjugate produced by the method shown in the aforementioned (1-6) as it is, or can be produced by purifying same and dissolving same in a solvent mainly containing water and approximately isotonic with the living body. In this case, the radiopharmaceutical is preferably in the form of an aqueous solution, and may contain other pharmaceutically acceptable components as necessary. An effective amount of the radiopharmaceutical is orally or parenterally, for example, intravenously, subcutaneously, intraperitoneally, intramuscularly, or the like, administered to a living body, and is used for treatment of cancer, diagnosis of cancer, detection of a lesion, or the like.

As used herein, the subject of administration is a human, or an animal such as mouse, rat, monkey, guinea pig, chimpanzee, sheep, goat, dog, cat, swine, bovine, horse or the like, but is not particularly limited. Preferred is a human.

A preferred target disease is cancer that overexpresses HER2. The type of HER2-overexpressing cancer to be treated, diagnosed or detected in the present invention is not particularly limited as long as it overexpresses HER2. Examples include salivary gland cancer, ovarian cancer, bladder cancer, biliary tract cancer, gastric cancer, and breast cancer. The HER2-overexpressing cancer may also be cancer of any stage, and may be localized or metastatic, or primary or recurrent. As used herein, the "overexpression" refers to a state in which, when measured by a known test

method, significant amplification of the HER2 gene in tumor tissue compared to non-tumor tissue, or significant enhancement of HER2 protein expression compared to non-tumor tissue is observed.

As used herein, the "effective amount" is an amount that can afford useful diagnostic or therapeutic effects in a subject of administration. The effective amount to be administered to a subject varies depending on the type of subject, body weight of the subject, dosage form (tablet, injection, etc.) and route (oral administration, parenteral administration, etc.) of administration, severity of disease (e.g., cancer), and the like. Physicians and veterinarians can consider these factors and determine the appropriate effective amount.

The radiopharmaceutical of the present invention when stored at room temperature has a radiochemical purity of a certain level or above at the time of expiry of a period that is a multiple of not less than 1 and not more than 5 of the half-life, based on the half-life of the metal radionuclide constituting the radiopharmaceutical. When the above-mentioned metal radionuclide is a β-ray nuclide (e.g., Lu-177 or Y-90), the radiochemical purity of the conjugate is preferably not less than 90%, more preferably not less than 95%, when stored at room temperature for 7 days after production. When the metal radionuclide is an α-ray nuclide (e.g., Ac-225), the radiochemical purity of the conjugate after storage for 14 days at room temperature from the completion of the production is preferably not less than 90%, more preferably not less than 95%. The "room temperature" in the present specification preferably refers to the "ordinary temperature" defined in the Japanese Pharmacopoeia, which is specifically 15 to 25°C.

As used herein, the radiochemical purity refers to the percentage of the peak radioactivity (count) corresponding to the conjugate with respect to the total radioactivity (count) detected when the sample is analyzed with a commercially available radiation detector. High performance liquid chromatography and thin-layer chromatography can be used for the analysis of radiochemical purity, and thin-layer chromatography is preferably used. More preferably, thin-layer chromatography is used under the conditions described in the below-mentioned Examples.

As described above, the radiopharmaceutical of the present invention is preferably in the form of an aqueous solution. It is more preferably in the form of a buffer from the aspect of maintaining the radiochemical purity as described above. As the buffer, any buffer used in an antibody drug containing ADC of an anti-HER2 antibody or an anti-HER2 antibody as an active ingredient can be used. As a nonlimiting example, histidine buffer or succinate buffer can be used. The histidine buffer is composed of histidine and a salt thereof, for example, histidine and hydrochloride thereof or histidine and acetate thereof. The succinate buffer is composed of succinic acid and a salt thereof, for example, succinic acid and a sodium salt thereof. The radiopharmaceutical of the present invention may contain any sugar such as sucrose or trehalose, and may also contain a solubilizer such as polysorbate 20 and polysorbate 80.

The radiopharmaceutical of the present invention can be used for radionuclide therapy of cancer by selecting metal radionuclides that have a therapeutic effect, specifically radionuclide that emits α rays or nuclide that emits β rays (preferably Ac-225, Y-90, Lu-177, more preferably Ac-225). In this radionuclide therapy, the radiopharmaceutical of the present invention is administered by intravenous injection or orally to cause accumulation of the radioconjugate of the present invention in a lesion site such as a cancer primary lesion or metastatic lesion, and cancer cells at the lesion site are destroyed by the radiation emitted from the metal radionuclide. The amount to be administered and dose of the radiopharmaceutical of the present invention is appropriately determined by the efficacy of the active ingredient, the mode and route of administration, the stage of cancer progression, the body type, body weight and age of the patient, and the type and amount of the therapeutic drug used in combination for other diseases.

In addition, by selecting a radionuclide that emits positrons or a radionuclide that emits γ rays (preferably Zr-89) as the metal radionuclide, it can be used for cancer diagnosis or lesion detection. A radiopharmaceutical using radionuclide that emits positrons can be preferably used for PET (Positron Emission Tomography) examination, and a radiopharmaceutical using radionuclide that emits γ-rays can be preferably used for SPECT (Single Photon Emission Computed Tomography) examination. This may also be used in combination with cancer diagnosis or lesion detection in the aforementioned radionuclide therapy of cancer. The diagnostic radiopharmaceutical for cancer of the present invention may be used for diagnosis before performing radionuclide therapy for cancer, or may be used for diagnosis after performing radionuclide therapy for cancer. Using the radiopharmaceutical for diagnosis before conducting a radionuclide therapy for cancer, selection of treatment can be performed based on whether or not to perform radionuclide therapy for cancer using the radiopharmaceutical of the present invention provided with a metal nuclide that emits α-rays. Using the radiopharmaceutical for diagnosis after conducting a radionuclide therapy for cancer, moreover, whether or not radionuclide therapy for cancer using the radiopharmaceutical of the present invention is effective can be judged, and a treatment plan including an increase or a decrease of dose and the like can be optimized.

(2) Radiopharmaceutical (Radiopharmaceutical (2))

Another embodiment of the present invention is a radiopharmaceutical containing a conjugate of a chelating agent chelated with a metal radionuclide and an anti-HER2 antibody as an active ingredient, wherein the linkage between the anti-HER2 antibody and the chelating agent does not contain a thiourea bond, when stored at room temperature, it has a radiochemical purity of a certain level or above at the time of expiry of a period that is a multiple of not less than

1 and not more than 5 of the half-life, based on the half-life of the metal radionuclide constituting the radiopharmaceutical. When the above-mentioned metal radionuclide is a β-ray nuclide (e.g., Lu-177 or Y-90), the radiochemical purity of the aforementioned conjugate is preferably not less than 90%, more preferably not less than 95%, when stored at room temperature for 7 days after production. Also, when the metal radionuclide is an α-ray nuclide (e.g., Ac-225), the radiochemical purity of the conjugate is preferably not less than 90%, more preferably not less than 95%, when stored at room temperature for 14 days after production. The room temperature is as defined in the aforementioned radiopharmaceutical (1).

In the radiopharmaceutical (2), the following methods (a) to (d) can also be used in conjugating the chelating agent and the anti-HER2 antibody, in addition to the site-specific modification method using a peptide. Since other part is the same as in the radiopharmaceutical (1), the explanation is omitted.

(a) method for modifying a sulfhydryl (SH) group generated by partially reducing a disulfide bond (SS bond) between polypeptide chains at the hinge site of an antibody with a chelating agent or linker (L) having a maleimide group reactive with the SH group
(b) method for modifying cysteine newly introduced into an antibody by an amino acid mutation by genetic engineering with a chelating agent or linker (L) having a maleimide group
(c) method for modifying an azide group of azidized lysine newly introduced into an antibody by an amino acid mutation by genetic engineering with a chelating agent or linker (L) having alkyne (e.g., Dibenzocyclooctyne: DBCO) by using a click reaction
(d) method for modifying glutamine introduced into a specific position of an antibody with a chelating agent or linker (L) having a side chain of lysine by using transglutaminase

In the present invention, a peptide that site-specifically modifies an anti-HER2 antibody and a chelating agent are linked without using a thiourea bond. Therefore, a radioconjugate and a radiopharmaceutical that are stable even at room temperature can be obtained. Since the site-specific modification of antibody can contain monovalent antibody or divalent antibody or both of these in any proportion, unlike random modification, radioconjugate and radiopharmaceutical with stable quality can be obtained. In addition, the radioconjugate of the present invention maintains efficacy equivalent to conventional one. Therefore, according to the present invention, an anti-HER2 antibody conjugate and a radiopharmaceutical thereof with higher quality while maintaining efficacy can be provided.

The following embodiments are also encompassed in the technical idea of the present invention.

[1] A conjugate of an anti-HER2 antibody site-specifically modified with a peptide and a chelating agent, wherein the aforementioned chelating agent is chelated with a metal radionuclide, the aforementioned peptide and the chelating agent are linked by a linker (L), and the aforementioned linker (L) does not contain a thiourea bond.
[2] The conjugate of [1], wherein the aforementioned chelating agent is DOTAGA (α-(2-Carboxyethyl)-1,4,7,10-tetraazacyclododecane-1,4,7,10-tetraacetic acid).
[3] The conjugate of [1] or [2], wherein the aforementioned peptide is an amino acid sequence consisting of not less than 13 and not more than 17 amino acid residues and is represented by the following formula (i):

(Xa)-Xaa1-(Xb)-Xaa2-(Xc)-Xaa3-(Xd)··· (i)

in the formula (i), Xa, Xb, Xc and Xd are continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy $a+b+c+d \leq 14$,
Xaa1 and Xaa3 are each independently an amino acid residue derived from an amino acid having a thiol group in the side chain, or an amino acid residue derived from an amino acid having a haloacetyl group in the side chain, provided that one of Xaa1 and Xaa3 is an amino acid residue derived from an amino acid having a thiol group in the side chain,
Xaa1 and Xaa3 are connected to form a ring structure, and
Xaa2 is a lysine residue, an arginine residue, a cysteine residue, an aspartic acid residue, a glutamic acid residue, 2-aminosuberic acid, or diamino propionic acid, and modified with a crosslinking agent.

[4] The conjugate of any one of [1] to [3], wherein the aforementioned metal radionuclide is Ac-225, Y-90, Lu-177, or Zr-89.
[5] The conjugate of any one of [1] to [4], wherein the aforementioned linker (L) comprises the formula (10a), the formula (10b), or the formula (10c):

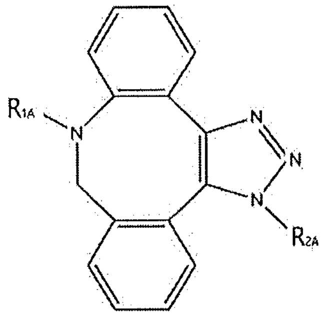

(10a)

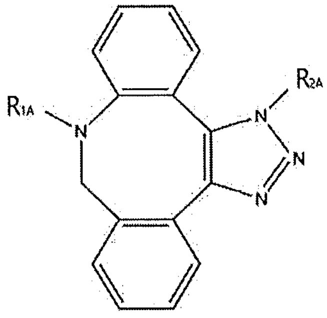

(10b)

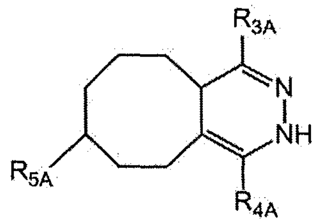

(10c)

In the formula (10a) and the formula (10b), $R_{1A}$ is a binding site with a chelating agent, and $R_{2A}$ is a binding site with the aforementioned peptide. In the formula (10c), one of $R_{3A}$ and $R_{4A}$ is a hydrogen atom, a methyl group, a phenyl group or a pyridyl group, and the other is a binding site with the aforementioned chelating agent, and $R_{5A}$ is a binding site with the aforementioned peptide.

[6] The radioconjugate of [5], comprising a polyethylene glycol group between the linkage site with the aforementioned peptide and the aforementioned peptide.

[7] The radioconjugate of any one of [1] to [6], which is conjugated by a click reaction of an anti-HER2 antibody site-specifically modified with the aforementioned peptide having an azide group introduced into the N-terminal, and a radioactive metal complex of DOTAGA-DBCO represented by the following formula:

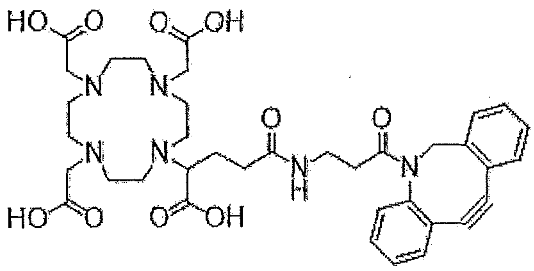

DOTAGA-DBCO

[8] The conjugate of any one of [1] to [7], wherein the aforementioned anti-HER2 antibody is trastuzumab.

[9] The conjugate of any one of [1] to [7], wherein the aforementioned anti-HER2 antibody is pertuzumab.

[10] A radiopharmaceutical comprising the conjugate of any one of [1] to [9] as an active ingredient.

[11] The radiopharmaceutical of [10], which is used in a radionuclide therapy for cancer.

[12] The radiopharmaceutical of [10], which is used in cancer diagnosis.

[13] The radiopharmaceutical of [12], which is used in combination with the radionuclide therapy for cancer using a radiopharmaceutical in [11].

[14] A radiopharmaceutical containing a conjugate of a chelating agent chelated with a metal radionuclide and an anti-HER2 antibody as an active ingredient, wherein the linkage between the anti-HER2 antibody and the chelating agent does not contain a thiourea bond, and the conjugate has a radiochemical purity of not less than 90% when stored at room temperature for 7 days.

[15] The radiopharmaceutical of [14], wherein the aforementioned conjugate is any one of [1] to [9].

[16] The radiopharmaceutical of [15], which is used in a radionuclide therapy for cancer.

[17] The radiopharmaceutical of [15], which is used in cancer diagnosis.

[18] The radiopharmaceutical of [17], which is used in combination with the radionuclide therapy for cancer using a radiopharmaceutical in [16].

[19] A radiopharmaceutical containing a conjugate of a chelating agent chelated with a metal radionuclide and an anti-HER2 antibody as an active ingredient and satisfying the following condition (1) or (2), wherein the linkage between the anti-HER2 antibody and the chelating agent does not contain a thiourea bond:

(1) the aforementioned metal radionuclide is $^{177}Lu$ or $^{90}Y$, and the aforementioned conjugate has a radiochemical purity of not less than 90% when stored at room temperature for 7 days

(2) the aforementioned metal radionuclide is $^{225}$Ac, and the aforementioned conjugate has a radiochemical purity of not less than 90% when stored at room temperature for 14 days.

[20] A radiopharmaceutical containing a conjugate of a chelating agent chelated with a metal radionuclide and an anti-HER2 antibody as an active ingredient, wherein the linkage between the anti-HER2 antibody and the chelating agent does not contain a thiourea bond, and the conjugate has a radiochemical purity of not less than 90% at the time of expiry of a period that is a multiple of not less than 1 and not more than 5 of the aforementioned half-life, based on the half-life of the aforementioned metal radionuclide.

[Example]

The present invention is described in more detail in the following by way of examples. However, the scope of the present invention is not limited by the examples. In the Tables below, the column with "-" indicates no performance.

[Example 1] Production of conjugate with trastuzumab by using $^{225}$Ac-labeled DOTAGA-DBCO

(1. Antibody modification step)

A peptide containing 17 amino acid residues represented by the following formula (P3) was obtained by the method described in WO 2017/217347. The amino acid sequence of this peptide was the same as the sequence in which Xaa2 of SEQ ID NO: (2) was a lysine residue, and the side chain terminal amino group of the lysine residue was modified with the structure shown by $R_1$. In addition, two cysteine residues form a disulfide bond with each other, and to the N-terminal of the peptide was added ethyl azide as an atomic group containing an azide group, which is the second atomic group, via a linker ($L_1$) structure having diglycolic acid and eight PEGs.

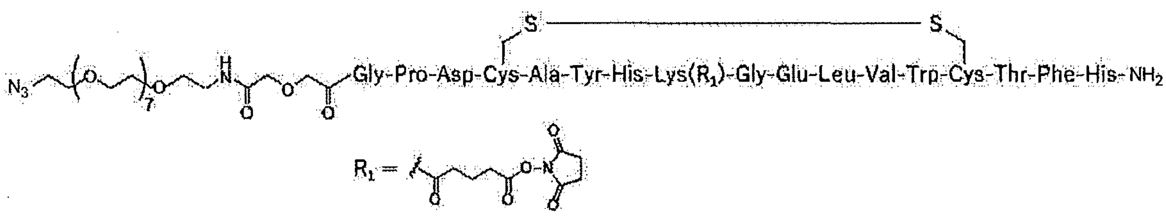

(P3)

(SEQ ID NO: 17)

In the formula (P3), Gly is glycine, Pro is proline, Asp is aspartic acid, Cys is cysteine, Ala is alanine, Tyr is tyrosine, His is histidine, Glu is glutamic acid, Leu is leucine, Val is valine, Trp is tryptophan, Phe is phenylalanine.

A mixture of the peptide and trastuzumab (herceptin (registered trade mark) manufactured by Roche) in a 0.02 mol/L sodium acetate buffer (pH 6.0) was reacted at room temperature for 30 min to give a solution containing a peptide-modified antibody. The peptide-modified antibody has an Fc region of the antibody site-specifically modified by the above-mentioned peptide.

The solution was then passed through the IgG-BP column to separate into the first antibody composition containing relatively large amounts of the unlabeled antibody and the monovalent antibody and the second antibody composition containing a relatively large amount of the divalent antibody.

A solution containing the peptide-modified antibody obtained in the above-mentioned step was diluted with 0.02 mol/L sodium acetate buffer (pH 6.0), added to the above-mentioned IgG-BP column, and a 0.10 mol/L sodium acetate buffer (pH 5.7) containing 0.15 mol/L sodium chloride was flown. A second antibody composition was recovered, and the concentration was adjusted such that the concentration of the divalent antibody contained in the recovered fraction was 15 mg/mL. Thereafter, 0.10 mol/L sodium acetate buffer (pH 3.5) containing 0.15 mol/L sodium chloride was flown through the IgG-BP column, a first antibody composition was recovered, and the concentration was adjusted such that the concentration of the monovalent antibody contained in the recovered fraction was 15 mg/mL. A solution containing the obtained first antibody composition was subjected to the below-mentioned labeling step.

(2. Complex formation step)

DOTAGA-DBCO represented by the following formula was produced based on the method described in Bernhard et al. DOTAGA-Anhydride: A Valuable Building Block for the Preparation of DOTA-Like Chelating Agents Chem.

Eur. J. 2012, 18, 7834-7841. This chelating agent was dispersed in 0.1 mol/L sodium acetate buffer (pH 6.0) as a solvent to give a dispersion containing 1.7 mmol/L chelating agent. A reaction mixture of the dispersion (0.005 mL), 0.1 mol/L sodium acetate buffer (pH 6.0, 0.075 mL), and $^{225}$Ac ion-containing solution (0.2 mol/L aqueous hydrochloric acid solution, radioactivity concentration 320 - 340 MBq/mL, prepared from one produced by Oak Ridge National Laboratory, liquid amount 0.005 mL) 1.6 to 1.7 MBq (calculated by attenuation from the level of radioactivity at test date and time) as a radioactive metal source was reacted under heating conditions to give a $^{225}$Ac complex solution. The molar ratio of the chelating agent and the radioactive metal ion was chelating agent:$^{225}$Ac ion = about 2500:1, and the heating temperature of the reaction mixture was set to 70°C, and the heating time was set to 30 min.

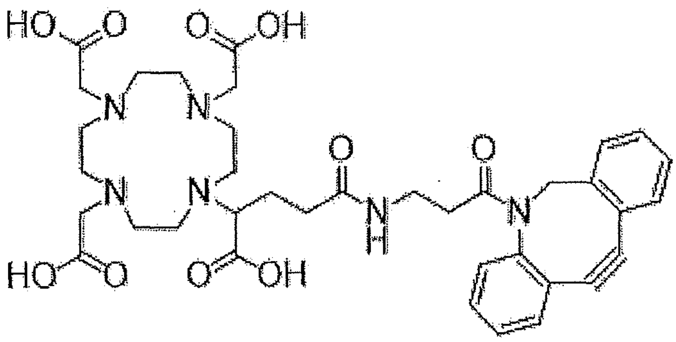

DOTAGA-DBCO

The radiochemical purity (RCP) of the obtained $^{225}$Ac complex was measured by the following method. That is, a part of the $^{225}$Ac complex solution was developed by thin layer chromatography (manufactured by Agilent, model number: SGI0001, eluent: acetonitrile/water mixed solution (volume ratio 1:1)), and then measured by radio γ-TLC Analyzer (manufactured by raytest, MODEL GITA Star). The percentage of the radioactivity (count) of the peak detected near the origin with respect to the detected total radioactivity (count) was defined as the RCP (%) of the $^{225}$Ac complex. As a result, the RCP of the $^{225}$Ac complex was 90%. The obtained $^{225}$Ac complex solution was directly used for the labeling step.

(3. Labeling step)

A solution of the unpurified $^{225}$Ac complex obtained via the aforementioned step (2), and a solution containing a peptide-modified antibody (monovalent antibody) obtained in the above-mentioned step (1) were each added to 0.1 mol/L arginine-containing 0.1 mol/L histidine buffer (pH 6.0), and a click reaction was performed at 37°C for 120 min to give $^{225}$Ac complex-labeled antibody. The amount of the $^{225}$Ac complex and the amount of the peptide-modified antibody (monovalent antibody) were each 85 nmol, and the molar ratio of the DBCO group and the azide group was about 1:1.2. The reaction rate (%) of the unpurified $^{225}$Ac complex-labeled antibody is shown in the following Table 1. Here, the reaction rate (%) means the RCP (%) of the $^{225}$Ac complex-labeled antibody with respect to the labeling rate (%) in the complex formation step, and the labeling rate (%) means the level of radioactivity (%) of the $^{225}$Ac complex with respect to the charged radioactivity level.

Furthermore, a solution of the $^{225}$Ac complex-labeled antibody obtained by reacting at 37°C for 2 hr was purified using ultrafiltration filter (manufactured by Merck, model number: UFC505096). The RCP and the radiochemical yield (RCY) of the $^{225}$Ac complex-labeled antibody after purification are shown in the following Table 1.

The measurement method of the RCP and RCY of the $^{225}$Ac complex-labeled antibody was as follows. That is, thin layer chromatography (manufactured by Agilent, model number: SGI0001, developing solvent was mixed solution of acetonitrile:0.1 mmol/L EDTA solution (volume ratio 1:1)) was measured by radio γ-TLC Analyzer (manufactured by raytest, MODEL GITA Star), and the percentage of the radioactivity (count) of the peak detected near the origin to the total radioactivity (count) detected was defined as the RCP (%). In addition, the percentage of the radioactivity (radioactivity level calculated from the count measured by γ ray spectrometer (Ge semiconductor detector: GMX10P4-70 (manufactured by ORTEC), Multi Channel Analyzer: M7-000 (manufactured by SEIKO EG&G), data processing: Spectrum Navigator:DS-P300 (manufactured by SEIKO EG&G) and Gamma Studio:DS-P600 (manufactured by SEIKO EG&G)) recovered after ultrafiltration purification with respect to the total radioactivity (similar to the above, the radioactivity level calculated from the count measured by γ ray spectrometer) added at the start of the labeling step was defined as the RCY (%).

[Table 1]

| | chelating agent (A) | antibody (B) | molar ratio (A):(B) | reaction rate | after purification | |
|---|---|---|---|---|---|---|
| | | | | 37°C 2 hr reaction (%) | RCP (%) | RCY (%) |
| Example 1 | DOTAGA | trastuzumab | 1:1.2 | 77% | 93% | 71% |

[Example 2] Production of conjugate with trastuzumab by using $^{89}Zr$-labeled DOTAGA-DBCO

(1. Complex formation step)

DOTAGA-DBCO was dispersed in DMSO as a solvent to give a dispersion containing 0.33 mmol/L chelating agent. A reaction mixture of the dispersion (0.030 mL), and $^{89}Zr$ ion-containing solution (0.1 mol/L aqueous hydrochloric acid solution, radioactivity concentration 181 MBq/mL, prepared from one manufactured by Nihon Medi-Physics Co., Ltd., liquid amount 0.33 mL) 60 MBq as a radioactive metal source was reacted under heating conditions to give a $^{89}Zr$ complex solution. The molar ratio of the chelating agent and the radioactive metal ion was chelating agent:$^{89}Zr$ ion = about 250:1, and the heating temperature of the reaction mixture was set to 70°C, and the heating time was set to 60 min.

The RCP of the obtained $^{89}Zr$ complex was measured by the following method. That is, a part of the $^{89}Zr$ complex solution was developed by thin layer chromatography (manufactured by Agilent, model number: SGI0001, developing solvent: acetonitrile/water mixed solution (volume ratio 1:1)), and then measured by radio γ-TLC Analyzer (manufactured by raytest, MODEL GITA Star PS). The percentage of the radioactivity (count) of the peak detected near the origin with respect to the detected total radioactivity (count) was defined as the RCP (%) of the $^{89}Zr$ complex. As a result, the RCP of the $^{89}Zr$ complex was 90%. The obtained $^{89}Zr$ complex solution was used as it was in the labeling step.

(2. Labeling step)

A solution of the unpurified $^{89}Zr$ complex obtained in the aforementioned step (1), and a solution containing a peptide-modified antibody (monovalent antibody) obtained in the same manner as in Example 1 were each added without purification to 0.1 mol/L arginine-containing 0.1 mol/L histidine buffer (pH 6.0), and a click reaction was performed at 37°C for 120 min to give $^{89}Zr$ complex-labeled antibody of Example 2. The amount of the $^{89}Zr$ complex and the amount of the peptide-modified antibody (monovalent antibody) were each 100 nmol, and the molar ratio of DBCO and azide was each about 1:1. The reaction rate (%) of the unpurified $^{89}Zr$ complex-labeled antibody of the Example is shown in the following Table 2. Here, the reaction rate (%) means RCP(%) of the $^{89}Zr$ complex-labeled antibody with respect to the labeling rate (%) in the complex formation step, and the RCP (%) means the amount of radioactivity (%) of the $^{89}Zr$ complex with respect to the charged radioactivity amount.

Furthermore, a solution of the $^{89}Zr$ complex-labeled antibody obtained by reacting at 37°C for 2 hr was purified using ultrafiltration filter (manufactured by Merck, model number: UFC505096). The RCP and RCY of the $^{89}Zr$ complex-labeled antibody after purification are shown in the following Table 2.

The measurement method of the RCP and RCY of the $^{89}Zr$ complex-labeled antibody was similar to that in Example 1.

[Table 2]

| | chelating agent (A) | antibody (B) | molar ratio (A):(B) | reaction rate | after purification | |
|---|---|---|---|---|---|---|
| | | | | 37°C 2 hr reaction (%) | RCP(%) | RCY (%) |
| Example 2 | DOTAGA | trastuzumab | 1:1 | 52% | 93% | 43% |

[Comparative Example 1] Production of conjugate with trastuzumab by using $^{225}Ac$-labeled DOTA-DBCO

The operation was performed according to Example 1 except that DOTAGA-DBCO was changed to the following DOTA-DBCO. The results are shown in Table 3.

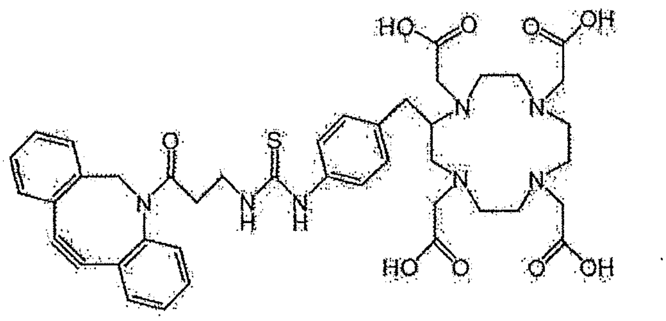

DOTA-DBCO

[Table 3]

| | chelating agent (A) | antibody (B) | molar ratio (A):(B) | reaction rate | after purification | |
|---|---|---|---|---|---|---|
| | | | | 37°C 2 hr reaction (%) | RCP (%) | RCY (%) |
| Comparative Example 1 | DOTA | trastuzumab | 1:1 | 60% | 95% | 34% |

[Example 3] Formulation step

A portion of each of the radioconjugates prepared as described in Example 1 and Comparative Example 1 was placed in a 0.5 mL Eppen tube (LoBind, manufactured by Eppendorf) and diluted with a storage buffer (19 g/L trehalose hydrate, 0.47 g/L L-histidine hydrochloride hydrate, 0.30 g/L L-histidine, and 85 mg/L polysorbate 20 mixed solution).

[Evaluation 1] Stability evaluation

Each radioconjugate obtained in Example 3 was stored at room temperature (24.5-25.5°C) for 2 weeks, and RCP, proportion of aggregates, and antigen binding activity were evaluated at each time point (0 day point, 1 day point, 7 day point, and/or 14 day point). Note that 14 days after the end of production corresponds to about 1.5 half-life when the metal radionuclide is Ac-225.

[Evaluation 1-1] Radiochemical purity

RCP was analyzed by thin layer chromatography (TLC). The TLC conditions were the same as those used in Example 1 to examine the reaction rate.

The results are shown in Table 4.

[Table 4]

| | radiochemical purity (%) | | | |
|---|---|---|---|---|
| | 0 day point | 1 day point | 7 day point | 14 day point |
| radioconjugate (Example 1) | 99.98 | 99.91 | 99.93 | 99.84 |
| radioconjugate (Comparative Example 1) | 98.32 | 98.74 | 94.66 | 76.54 |

The radioconjugate prepared as described in Example 1 containing no thiourea bond maintained an RCP of 99% or more when stored at room temperature for 7 days after completion of the production. Even when stored at room temperature for 14 days after completion of the production, 99% or more of RCP was maintained.

The radioconjugate prepared as described in Comparative Example 1 containing a thiourea bond maintained an RCP of 90% or more but below 95% when stored at room temperature for 7 days after completion of the production.

When stored at room temperature for 14 days after completion of the production, the RCP was less than 80%.

[Evaluation 1-2] Proportion of aggregate

The proportion of aggregates was confirmed by size-exclusion chromatography (SEC). Using Model 2695 Separation Module or e2695 Separation Module, manufactured by Waters, as a liquid chromatography device, and Model 2489 UV/Vis Detector manufactured by Waters as a UV detector, analysis was performed under the following conditions. The proportion of each component when stored for 14 days after completion of the production is shown in Table 5. When stored for 14 days after completion of the production, the proportion of aggregates of the radioconjugates prepared as described in Example 1 and containing no thiourea bond was lower than that of the aggregates of the radioconjugates prepared as described in Comparative Example 1 and containing a thiourea bond.

[Table 5]

| | proportion of main peak (%) | proportion of aggregate peak (%) |
|---|---|---|
| radioconjugate (Example 1) | 94.79 | 1.51 |
| radioconjugate (Comparative Example 1) | 93.18 | 3.34 |

[HPLC conditions]

column: TOSOH TSKgel guard column SWXL (6 mm × 4cm), TOSOH TSKgel G3000SWXL (5 μm, 7.8×30 cm)× 2 (tandem)
column temperature: constant temperature around 25°C
mobile phase: 0.2 mol/L arginine hydrochloride-containing 0.1 mol/L phosphate buffer (pH 6.8)
flow: 1.0 mL/min
area measurement range: 30 min
detection wavelength: 280 nm

[Evaluation 1-3] Antigen binding activity

The antigen binding activity was confirmed by in vitro autoradiography (ARG) (production day (0 day point) and last day of storage (14 day point) alone). SK-OV-3 cells of HER2-positive human ovarian cancer cell line and MDA-MB-231 cells of HER2-negative human breast cancer cell line, which were purchased from ATCC (American Type Culture Collection), were administered subcutaneously to the flanks of female BALB/c nu/nu mice at $5\times10^6$ cells and $1\times10^7$ cells, respectively, to prepare tumor-bearing mice. Thereafter, SK-OV-3 tumor and MDA-MB-231 tumor were excised and embedded in Tissue-Tek O.C.T. Compound (Japanese Sakura Finetek Japan Co., Ltd.) to prepare frozen sections. The radioconjugates obtained in Example 1 and Comparative Example 1 were added to 10% bovine serum albumin-containing PBS each at 1 kBq/mL, and SK-OV-3 tumor section and MDA-MB-231 tumor section were immersed therein. After contacting the sections with the imaging plate, they were read with a scanner-type image analyzer to evaluate the level of radioactivity bound to the sections. The results are shown in Fig. 3.

By performing the same evaluation of each solution with trastuzumab added thereto, the specificity of each radioconjugate for HER2 can be confirmed.

At the end point of storage (14 day point), binding activity to HER2 was confirmed in all of the radioconjugates prepared as described in Example 1 and Comparative Example 1. Both radioconjugates prepared as described in Example 1 and Comparative Example 1 bound only to SK-OV-3 tumor section, demonstrating HER2 selective binding. In the solution added with trastuzumab, binding to the SK-OV-3 tumor section was inhibited, and HER2 specificity of binding was confirmed. At the end point of storage (14 day point), selectivity of binding to HER2 was maintained in all samples. The radioactivity bound to SK-OV-3 tumor section was higher in the sample using the radioconjugate prepaed as described in Example 1 than the radioconjugate prepared as described in Comparative Example 1.

[Evaluation 2] Efficacy evaluation

A subcutaneous tumor-bearing model of SK-OV-3 cells was prepared using a mouse, and the antitumor effect of the radioconjugates prepared as described in Example 1 and Comparative Example 1 was confirmed.

SK-OV-3 cells of HER2-positive human ovarian cancer cell line purchased from ATCC were suspended in

McCoy's 5A medium (Gibco) and administered subcutaneously to the flanks of 5-week-old female BALB/c nu/nu (Charles River Laboratories Japan, Inc.) at $5\times10^6$ cells to prepare tumor-bearing mice. Three weeks after the tumor-bearing treatment, it was confirmed that the tumor volume was approximately 100 to 300 $mm^3$, and individuals with a shape suitable for tumor diameter measurement were randomly grouped. The tumor volume and body weight of each mouse at that time are shown in Table 6. The tumor volume was calculated according to the following formula.

$$\text{tumor volume } (mm^3) = (\text{tumor major axis} \times (\text{tumor minor axis})^2) \times 1/2$$

[Table 6]

| | tumor volume mean±standard deviation ($mm^3$) | body weight mean±standard deviation (g) |
|---|---|---|
| radioconjugate (Example 1) group | 218.1±38.1 | 19.8±1.6 |
| radioconjugate (Comparative Example 1) group | 212.6±34.3 | 20.0±1.2 |
| antibody control group | 210.0128.7 | 19.3±1.4 |
| Vehicle group | 207.6±32.6 | 20.0±1.4 |

The radioconjugates prepared as described in Example 1 and Comparative Example 1 were administered into the tail vein at a dose of 15 kBq/mouse (50 μg/mouse as trastuzumab). As a control group, a group administered with trastuzumab with the same amount of antibody as each radioconjugate (antibody control group) and a Vehicle group administered with a storage buffer were set. Each group contained 6 mice, and observation of general condition and measurement of the body weight and tumor volume were performed over time for 38 days after administration. The changes in tumor volume over time are shown in Fig. 1, and the change in body weight over time is shown in Fig. 2.

The groups administered with the radioconjugates prepared as described in Example 1 and Comparative Example 1 showed a significant difference in the antitumor effect as compared with the two control groups (antibody control group and Vehicle group) at 38 day point after the administration ($P<0.01$). For determination of significant difference, Tukey test was performed using statistical analysis software Stat Preclinica (manufactured by Takumi Information Technology Inc.). On the other hand, no significant difference in antitumor effect was observed between the groups to which each radioconjugate was administered. In each group, no significant change was found in the general condition, and no sign of toxicity such as significant loss of weight was observed.

[Example 4] Production of conjugate with trastuzumab by using $^{177}Lu$-labeled DOTAGA-DBCO

(1. Antibody modification step)

This step was performed by a method similar to the method described in the antibody modification step in Example 1.

(2. Complex formation step)

DOTAGA-DBCO was produced in the same manner as in Example 1. This chelating agent was dispersed in 0.156 mol/L sodium acetate buffer (pH 5.5) as a solvent to give a dispersion containing 0.45 mmol/L chelating agent. A reaction mixture of the dispersion (0.02 mL), 0.156 mol/L sodium acetate buffer (pH 5.5, 0.02 mL) dissolving 0.225 mmol/L gentisic acid, and $^{177}Lu$ ion-containing solution (0.04 mol/L aqueous hydrochloric acid solution, radioactivity concentration 516 MBq/mL, prepared from one produced by POLATOM, liquid amount 0.05 mL) 25.8 MBq (calculated by attenuation from the amount of radioactivity at test date and time) as a radioactive metal source was reacted under heating conditions to give a $^{177}Lu$ complex solution. The molar ratio of the chelating agent and the radioactive metal ion was chelating agent: $^{177}Lu$ ion = about 236:1, and the heating temperature of the reaction mixture was set to 70°C, and the heating time was set to 5 min.

The RCP of the obtained $^{177}Lu$ complex was measured in the same manner as in the measurement of RCP

of the radioconjugate of Example 1. As a result, the RCP of the $^{177}$Lu complex was 99%. The obtained $^{177}$Lu complex solution was directly used for the labeling step.

(3. Labeling step)

A solution of the unpurified $^{177}$Lu complex obtained in the aforementioned step (2), and a solution containing a peptide-modified antibody (monovalent antibody) obtained in the above-mentioned step (1) were each added to 0.1 mol/L arginine-containing 0.1 mol/L histidine buffer (pH 6.0), and a click reaction was performed at 37°C for 120 min to give $^{177}$Lu complex-labeled antibody. The amount of the $^{177}$Lu complex and the amount of the peptide-modified antibody (monovalent antibody) were 8.4 nmol and 10 nmol, respectively, and the molar ratio of the DBCO group and the azide group was about 1:1.2. The reaction rate (%) of the unpurified $^{177}$Lu complex-labeled antibody is shown in the following Table 7.

In addition, the RCP and RCY of the $^{177}$Lu complex-labeled antibody after purification using an ultrafiltration filter in the same manner as in Example 1 are shown in the following Table 7.

The RCP and RCY of the $^{177}$Lu complex-labeled antibody were measured in the same manner as in Example 1.

[Table 7]

| | chelating agent (A) | antibody (B) | molar ratio (A):(B) | reaction rate | after purification | |
|---|---|---|---|---|---|---|
| | | | | 37°C 2 hr reaction (%) | RCP (%) | RCY (%) |
| Example 4 | DOTAGA | trastuzumab | 1:1.2 | 55% | 99% | 55% |

[Comparative Example 4] Production of conjugate with trastuzumab by using $^{177}$Lu-labeled DOTA-DBCO

The operation was performed according to Example 4 except that DOTAGA-DBCO was changed to DOTA-DBCO. The results are shown in Table 8.

[Table 8]

| | chelating agent (A) | antibody (B) | molar ratio (A):(B) | reaction rate | after purification | |
|---|---|---|---|---|---|---|
| | | | | 37°C 2 hr reaction (%) | RCP (%) | RCY (%) |
| Comparative Example 4 | DOTA | trastuzumab | 1:1.2 | 81% | 94% | 74% |

[Example 5] Production of conjugate with trastuzumab by using $^{90}$Y-labeled DOTAGA-DBCO

(1. Antibody modification step)

This step was performed by a method similar to the method described in the antibody modification step in Example 1.

(2. Complex formation step)

DOTAGA-DBCO was produced in the same manner as in Example 1. This chelating agent was dispersed in 0.156 mol/L sodium acetate buffer (pH 5.5) as a solvent to give a dispersion containing 0.3 mmol/L chelating agent. A reaction mixture of the dispersion (0.03 mL), 0.156 mol/L sodium acetate buffer (pH 5.5, 0.03 mL) dissolving 0.15 mmol/L gentisic acid, and $^{90}$Y ion-containing solution (0.04 mol/L aqueous hydrochloric acid solution, radioactivity concentration 3786 - 3943 MBq/mL, prepared from one produced by Eckert&Ziegler, liquid amount 0.03 mL) 113 to 118 MBq (calculated by attenuation from the amount of radioactivity at test date and time) as a radioactive metal source was reacted under heating conditions to give a $^{90}$Y complex solution. The molar ratio of the chelating agent and the radioactive metal ion was chelating agent:$^{90}$Y ion = 69 - 72:1, and the heating temperature of the reaction mixture was set to 70°C, and the heating time was set to 5 min.

The RCP of the obtained $^{90}$Y complex was measured in the same manner as in the measurement of RCP in

Example 1. As a result, the RCP of the $^{90}Y$ complex was 99%. The obtained $^{90}Y$ complex solution was directly used for the labeling step.

(3. Labeling step)

A solution of the unpurified $^{90}Y$ complex obtained in the aforementioned step (2), and a solution containing a peptide-modified antibody (monovalent antibody) obtained in the above-mentioned step (1) were each added to 0.1 mol/L arginine-containing 0.1 mol/L histidine buffer (pH 6.0), and a click reaction was performed at 37°C for 120 min to give $^{90}Y$ complex-labeled antibody. The amount of the $^{90}Y$ complex and the amount of the peptide-modified antibody (monovalent antibody) were 9 nmol and 10 nmol, respectively, and the molar ratio of the DBCO group and the azide group was about 1:1.1. The reaction rate (%) of the unpurified $^{90}Y$ complex-labeled antibody is shown in the following Table 9.

In addition, the RCP and RCY of the $^{90}Y$ complex-labeled antibody after purification using an ultrafiltration filter in the same manner as in Example 1 are shown in the following Table 9.

The RCP and RCY of the $^{90}Y$ complex-labeled antibody were measured in the same manner as in Example 1.

[Table 9]

| | chelating agent (A) | antibody (B) | molar ratio (A):(B) | reaction rate | after purification | |
|---|---|---|---|---|---|---|
| | | | | 37°C<br>2 hr reaction (%) | RCP (%) | RCY (%) |
| Example 5 | DOTAGA | trastuzumab | 1:1.1 | 88% | 99% | 77% |

[Comparative Example 5] Production of conjugate with trastuzumab by using $^{90}Y$-labeled DOTA-DBCO

The operation was performed according to Example 5 except that DOTAGA-DBCO was changed to the DOTA-DBCO. The results are shown in Table 10.

[Table 10]

| | chelating agent (A) | antibody (B) | molar ratio (A):(B) | reaction rate | after purification | |
|---|---|---|---|---|---|---|
| | | | | 37°C<br>2 hr reaction (%) | RCP (%) | RCY (%) |
| Comparative Example 5 | DOTA | trastuzumab | 1:1.2 | 57% | 92% | 45% |

[Example 6] Preservation stability of radioconjugate prepared as described in Example 4, Example 5, Comparative Example 4, or Comparative Example 5

(Formulation step)

A portion of each of the radioconjugates prepared as described in Example 4, Example 5, Comparative Example 4, or Comparative Example 5 was placed in a 0.5 mL Eppen tube (LoBind, manufactured by Eppendorf) and diluted with a storage buffer (42 g/L trehalose hydrate, 0.47 g/L L-histidine hydrochloride hydrate, 0.30 g/L L-histidine, and 85 mg/L polysorbate 20 mixed solution).

[Evaluation 3] Stability evaluation

Each radioconjugate obtained in Example 6 was stored at room temperature (20.6 - 21.8°C) for 2 weeks, and RCP and antigen binding activity were evaluated at each time point (0 day point, 1 day point, 7 day point, and/or 14 day point). Note that 7 days after the end of production corresponds to about 1 half-life when the metal radionuclide is Lu-177 and about 2.5 half-life when the metal radionuclide is Y-90. Also, 14 days after the end of production corresponds to about 2 half-life when the metal radionuclide is Lu-177 and about 5 half-life when the metal radionuclide is Y-90.

[Evaluation 3-1] Radiochemical purity

RCP was analyzed by TLC. The conditions of TLC were similar to those used for examining the reaction rate in Example 1. The results are shown in Table 11.

[Table 11]

| | radiochemical purity (%) | | | |
|---|---|---|---|---|
| | 0 day point | 1 day point | 7 day point | 14 day point |
| radioconjugate (Example 4) | 98.5 | 98.2 | 97.9 | 98.0 |
| radioconjugate (Comparative Example 4) | 96.7 | 95.4 | 84.7 | 59.8 |
| radioconjugate (Example 5) | 99.0 | 98.3 | 96.6 | 95.4 |
| radioconjugate (Comparative Example 5) | 92.2 | 87.7 | 72.8 | 62.4 |

The radioconjugate prepared as described in Example 4 or 5 containing no thiourea bond maintained an RCP of 95% or more when stored for 7 days after completion of the production. The radioconjugate prepared as described in Example 4 or 5 containing no thiourea bond maintained an RCP of 90% or more when stored for 14 days after completion of the production.

When the radioconjugate prepared as described in Comparative Example 4 was stored for 7 days at room temperature after completion of the production, RCP was below 85%, and when the radioconjugate prepared as described in Comparative Example 5 was stored for 7 days at room temperature after completion of the production, RCP was below 75%. When the radioconjugate prepared as described in Comparative Example 4 was stored for 14 days at room temperature after completion of the production, RCP was below 60%, and when the radioconjugate prepared as described in Comparative Example 5 was stored for 14 days at room temperature after completion of the production, RCP was below 65%.

[Evaluation 3-2] Antigen binding activity

The antigen binding activity was confirmed by in vitro ARG (0 and 14 day points alone). The evaluation was performed by a method similar to the method described in Evaluation 1-3. The results are shown in Figs. 4, 5.

At the end point of storage (14 day point), binding activity to HER2 was confirmed in all of the radioconjugates prepared as described in Example 4, Example 5, Comparative Example 4, or Comparative Example 5. All radioconjugates prepared as described in Example 4, Example 5, Comparative Example 4, or Comparative Example 5 only to SK-OV-3 tumor section, demonstrating HER2 selective binding. At the end point of storage (14 day point), selectivity of binding to HER2 was maintained in all samples. The radioactivity bound to SK-OV-3 tumor section was higher in the samples using the radioconjugates prepared as described in Examples 4 or 5 than in the samples using the radioconjugates prepared as described in Comparative Examples 4 or 5.

[Evaluation 4]

A subcutaneous tumor-bearing model of SK-OV-3 cells was prepared using mice, and the tumor accumulation of the radioconjugate prepared as described in Example 2 was confirmed.

SK-OV-3 cells of HER2-positive human ovarian cancer cell line purchased from ATCC were suspended in McCoy's 5A medium (Gibco) and administered subcutaneously to the flanks of 5-week-old BALB/c nu/nu (Charles River Laboratories Japan, Inc.) at $5\times10^6$ cells to prepare tumor-bearing mice. Four weeks after the tumor-bearing treatment, it was confirmed that the tumor volume was approximately 100 to 400 $mm^3$.

The radioconjugate prepared as described in Example 2 was administered at a dose of 5 MBQ/mouse (n=24) into the tail vein. After 60 hours from the administration, images were taken under the conditions of the following Table and using a small animal PET imaging device (PET/CT Si78, manufactured by Bruker).

Representative examples of the results of the PET imaging are shown in Fig. 6. A higher level of radioactivity was accumulated in the tumor as compared with other organs, and HER2 positive tumor could be depicted.

[Table 12]

| | |
|---|---|
| Isotope | 89-Zr |
| Acquisition time | 600 sec |
| Energy Window | 30% (357.7-664.3keV) |
| PET image reconstruction | MLEM GPU 32x32 0.25 (Iterations: 12) |
| correction | Scatter, Randoms, Decay, Partial volume, Attenuation |

[Example 7] Efficacy comparison between $^{225}$Ac complex-labeled antibody and ADC medicament

[Evaluation 5] Efficacy evaluation using $^{225}$Ac complex-labeled antibody

A subcutaneous tumor-bearing model of SK-OV-3 was prepared using a mouse, and the antitumor effect of the $^{225}$Ac complex-labeled antibody prepared as described in Example 1 and a commercially available Antibody-Drug-Conjugate (ADC) medicament was compared. As the ADC medicament, trastuzumab deruxtecan (ENHERTU (registered trade mark), Daiichi Sankyo Company, Limited) and trastuzumab emtansine (KADCYLA (registered trade mark), Chugai Pharmaceutical Co., Ltd.) were used. As an antibody control, trastuzumab (herceptin (registered trade mark), manufactured by Roche) was used.

The method described in Evaluation 2 was used to prepare SK-OV-3 tumor-bearing mouse. After the tumor-bearing treatment, it was confirmed that the tumor volume was 150 to 550 mm$^3$, and individuals with a shape suitable for tumor diameter measurement were randomly grouped. The tumor volume and body weight of the mice of each group at that time are shown in Table 13. ENHERTU (registered trade mark) was divided into low-dose administration group and high-dose administration group according to the doses. The low-dose administration group was adjusted so that the amount of the administered antibody was of the same level as in the $^{225}$Ac complex-labeled antibody group, and the high-dose administration group was adjusted so that the antibody was administered in an amount converted from the clinical dose in terms of mouse body weight.

[Table 13]

| | tumor volume mean±standard deviation (mm$^3$) | body weight mean±standard deviation (g) |
|---|---|---|
| radioconjugate (Example 1) administration group | 342.9157.9 | 19.8±1.2 |
| herceptin (registered trade mark) administration group | 302.7±123.8 | 20.7±1.1 |
| KADCYLA (registered trade mark) administration group | 270.2±23.4 | 20.3±1.8 |
| ENHERTU (registered trade mark) low dose administration group | 357.5±172.0 | 19.9±1.5 |
| ENHERTU (registered trade mark) high dose administration group | 379.1±113.7 | 20.9±1.3 |

The radioconjugate prepared as described in Example 1 was administered at a dose of 20 kBq/mouse (20 μg/mouse as trastuzumab) into the tail vein. In addition, herceptin (registered trade mark) was administered at a dose of 20 μg/mouse, KADCYLA (registered trade mark) was administered at a dose of 72 μg/mouse, and ENHERTU (registered trade mark) was administered at a dose of 20 μg/mouse in the low dose group and administered at a dose of 108 μg/mouse in the high dose group, into the tail vein. Each group contained 4 mice, and observation of general condition and measurement of the body weight and tumor volume were performed over time for 35 days after administration. The changes in tumor volume of the mice in each group are shown in Fig. 7.

The radioconjugate administration group showed a significant difference in the antitumor effect as compared with the ENHERTU (registered trade mark) low dose group at 35 day point after the administration ($P<0.05$). For determination of significant difference, Tukey test was performed using statistical analysis software Stat Preclinica. At 35 day point after the administration, moreover, it was confirmed that the antitumor effect tends to become stronger in the radioconjugate administration group as compared with ADC medicament administration groups other than the ENHERTU

(registered trade mark) low dose group, but no significant difference was found. In each group, no significant change was found in the general condition, and no sign of toxicity such as significant loss of weight was observed.

[Example 8] Efficacy comparison for each dose of radioconjugate [Evaluation 6] Efficacy evaluation using radioconjugate

A subcutaneous tumor-bearing model of SK-OV-3 was prepared using a mouse, and the antitumor effect of the radioconjugate prepared as described in Example 1 and commercially available ADC medicament was compared. As the ADC medicament, trastuzumab deruxtecan (ENHERTU (registered trade mark), Daiichi Sankyo Company, Limited) was used.

An SK-OV-3 tumor-bearing mouse was produced using the method described in Evaluation 2. Three weeks after the tumor-bearing treatment, it was confirmed that the tumor volume was 100 to 300 $mm^3$, and individuals with a shape suitable for tumor diameter measurement were randomly grouped. The tumor volume and body weight of each mouse at that time are shown in Table 14.

[Table 14]

| | tumor volume mean±standard deviation ($mm^3$) | body weight mean±standard deviation (g) |
|---|---|---|
| radioconjugate (Example 1) high radioactivity administration group | 210.5126.5 | 19.2±1.2 |
| radioconjugate (Example 1) moderate radioactivity administration group | 210.5128.8 | 18.0±1.0 |
| radioconjugate (Example 1) low radioactivity administration group | 205.4±33.6 | 19.0±1.8 |
| ADC medicament moderate dose administration group | 210.9±28.0 | 19.110.4 |
| antibody control group | 206.9±35.2 | 19.5±1.1 |
| Vehicle group | 209.7±30.9 | 18.8±1.1 |

The radioconjugate prepared as described in Example 1 was administered into the tail vein at a dose of 20 kBq/21 g in the high radioactivity administration group, at a dose of 10 kBq/21 g in the moderate radioactivity administration group, and at a dose of 5 kBq/21 g in the low radioactivity administration group (3.57 mg/kg as trastuzumab for each group). The ADC medicament was administered at a moderate dose of 10 mg/kg into the tail vein. In addition, a group administered with trastuzumab with the same amount of antibody (3.57 mg/kg as trastuzumab) as the radioconjugate administration group (antibody control group) and a Vehicle group administered with a storage buffer were set. Each group contained 6 mice, and observation of general condition and measurement of the body weight and tumor volume were performed over time for 46 days after administration. The changes in tumor volume of the mice in each group are shown in Fig. 8. On the final day of observation, the mice were autopsied, and the heart, lung, spleen, liver, and kidney were collected and weighed.

Each radioconjugate administration group showed a significant difference in the antitumor effect ($P<0.01$) at 46 day point after the administration as compared with the antibody control group and the Vehicle group. In addition, it was confirmed that the antitumor effect tends to become stronger depending on the administered radioactivity, suggesting that the antitumor effect becomes stronger depending on the administered radioactivity. For determination of significant difference, Tukey test was performed using statistical analysis software Stat Preclinica. On day 46 point of administration, no significant difference was found in the antitumor effect in the radioconjugate high radioactivity group and the radioconjugate moderate radioactivity group, as compared with the ADC medicament administration group, suggesting equivalent antitumor effects. In each group, no significant change was found in the general condition, and no sign of toxicity such as significant loss of weight was observed. On the last day of observation, no significant difference was observed in the weight of the organs collected by autopsy.

[Example 9] Production of conjugate with pertuzumab by using $^{225}$Ac-labeled DOTAGA-DBCO

(1. Antibody modification step)

A peptide represented by the above-mentioned formula (P3) containing an amino acid residue was obtained

by a method similar to that in Example 1.

A mixture of the peptide and pertuzumab (PERJETA (registered trade mark), Chugai Pharmaceutical Co., Ltd.) in a 0.02 mol/L sodium acetate buffer (pH 6.0) was reacted at room temperature for 60 min to give a solution containing a peptide-modified antibody. The peptide-modified antibody has an Fc region of the antibody site-specifically modified by the above-mentioned peptide.

The solution was then passed through the IgG-BP column to obtain the antibody composition containing relatively large amounts of the unlabeled antibody and the monovalent antibody. The concentration was adjusted with preservation buffer (41 g/L sucrose, 3.1 g/L L-histidine, 0.66 g/L glacial acetic acid mixed solution) (pH 6.0) such that the concentration of the monovalent antibody contained in the recovered fraction was 14.6 mg/mL. The obtained solution containing relatively large amounts of the unlabeled antibody and the monovalent antibody was subjected to the below-mentioned labeling step.

(2. Complex formation step)

DOTAGA-DBCO was produced in the same manner as in Example 1. This chelating agent was dispersed in 0.156 mol/L sodium acetate buffer (pH 5.5) as a solvent to give a dispersion containing 0.3 mmol/L chelating agent. A reaction mixture of the dispersion (0.02835 mL) and $^{225}$Ac ion-containing solution (0.1 mol/L aqueous hydrochloric acid solution, radioactivity concentration 259 MBq/mL, prepared from one produced by Rosatom State Atomic Energy Corporation, liquid amount 0.0126 mL) 3.25 MBq (calculated by attenuation from the level of radioactivity at test date and time) as a radioactive metal source was reacted under heating conditions to give a $^{225}$Ac complex solution. The molar ratio of the chelating agent and the metal radionuclide ion at that time was chelating agent:$^{225}$Ac ion = about 1270:1, and the heating condition of the reaction mixture was set to 70°C, heating time 30 min.

The RCP of the obtained $^{225}$Ac complex was measured in the same manner as in Example 1. As a result, the RCP of the $^{225}$Ac complex was 67%. The obtained $^{225}$Ac complex solution was directly used for the next labeling step.

(3. Labeling step)

A solution of the unpurified $^{225}$Ac complex obtained in the aforementioned step (2), and a solution containing a peptide-modified antibody (monovalent antibody) obtained in the above-mentioned step (1) were mixed and subjected to a click reaction at 37°C for 2 hr to give $^{225}$Ac complex-labeled antibody. The molar ratio of the DBCO group and the azide group was about 1:1.3. The reaction rate of the unpurified $^{225}$Ac complex-labeled antibody is shown in the following Table 15.

Furthermore, a solution of the $^{225}$Ac complex-labeled antibody obtained by reacting at 37°C for 2 hr was purified using ultrafiltration filter (manufactured by Merck, model number: UFC803096). The RCP and RCY of the $^{225}$Ac complex-labeled antibody after purification are shown in the following Table 15. The RCP and RCY of the $^{225}$Ac complex-labeled antibody were calculated based on the radioactivity level obtained by a method similar to that in Example 1.

[Table 15]

| | chelating agent (A) | antibody (B) | molar ratio (A):(B) | reaction rate (%) | after purification | |
|---|---|---|---|---|---|---|
| | | | | 37°C 2 hr reaction | RCP (%) | RCY (%) |
| Example 9 | DOTAGA | pertuzumab | 1:1.3 | 49 | 100 | 38 |

[Example 10] Formulated step

A portion of the radioconjugate prepared as described in Example 9 was placed in a 0.5 mL Eppen tube (LoBind, manufactured by Eppendorf) and diluted with a storage buffer (41 g/L sucrose, 3.1 g/L L-histidine, 0.66 g/L glacial acetic acid and 0.2 g/L polysorbate 20 mixed solution).

[Evaluation 7] Stability evaluation

The radioconjugate obtained in Example 9 was stored at room temperature (24.5-25.5°C) for 2 weeks, and RCP and the proportion of aggregates were evaluated at each time point (0 day point, 1 day point, 7 day point, and 14 day point).

[Evaluation 7-1] RCP

RCP was calculated from the TLC analysis results. The TLC conditions were the same as those used in Example 1 to evaluate the reaction rate. The results are shown in Table 16.

[Table 16]

| | RCP (%) | | | |
|---|---|---|---|---|
| | 0 day point | 1 day point | 7 day point | 14 day point |
| radioconjugate (Example 9) | 99.6 | 99.3 | 98.0 | 97.8 |

The radioconjugate prepared as described in Example 9 maintained an RCP of 98% or more when stored at room temperature for 7 days after completion of the production. Even when stored at room temperature for 14 days after completion of the production, 97% or more of RCP was maintained.

[Evaluation 7-2] Proportion of aggregate

The proportion of aggregates was confirmed by SEC, similar to the method described in Example 1. The proportion of each component at each evaluation day point when stored for 14 days after completion of the production is shown in Table 17. The proportion of aggregates when stored for 14 days after completion of the production was 1.66%.

[Table 17]

| | proportion of main peak (%) | proportion of aggregate peak (%) |
|---|---|---|
| radioconjugate (Example 9), 0 day point after production | 99.77 | 0.23 |
| radioconjugate (Example 9), 1 day point after production | 98.68 | 0.25 |
| radioconjugate (Example 9), 7 day point after production | 93.28 | 0.47 |
| radioconjugate (Example 9), 14 day point after production | 89.51 | 1.66 |

This application is based on a patent application No. 2020-174840 filed in Japan (filing date: October 16, 2020), a patent application No. 2020-215740 filed in Japan (filing date: December 24, 2020), and a patent application No. 2021-024688 filed in Japan (filing date: February 18, 2021), the contents of which are incorporated in full herein.

## Claims

**1.** A conjugate of an anti-HER2 antibody site-specifically modified with a peptide and a chelating agent, wherein

the chelating agent is chelated with a metal radionuclide, the peptide and the chelating agent are linked by a linker (L), and
the linker (L) does not contain a thiourea bond.

**2.** The conjugate according to claim 1, wherein the chelating agent is DOTAGA (α-(2-Carboxyethyl)-1,4,7,10-tetraazacyclododecane-1,4,7,10-tetraacetic acid).

**3.** The conjugate according to claim 1, wherein the peptide is an amino acid sequence consisting of not less than 13 and not more than 17 amino acid residues and is represented by the following formula:

(Xa)-Xaa1-(Xb)-Xaa2-(Xc)-Xaa3-(Xd)··· (i)

in the formula (i), Xa, Xb, Xc and Xd are continuous X in the number of a, continuous X in the number of b, continuous X in the number of c, and continuous X in the number of d, respectively,

X is an amino acid residue having neither a thiol group nor a haloacetyl group in the side chain,
a, b, c and d are each independently an integer of not less than one and not more than 5, and satisfy $a+b+c+d \leq 14$,
Xaa1 and Xaa3 are each independently an amino acid residue derived from an amino acid having a thiol group in the side chain, or an amino acid residue derived from an amino acid having a haloacetyl group in the side chain, provided that one of Xaa1 and Xaa3 is an amino acid residue derived from an amino acid having a thiol group in the side chain,
Xaa1 and Xaa3 are connected to form a ring structure, and
Xaa2 is a lysine residue, an arginine residue, a cysteine residue, an aspartic acid residue, a glutamic acid residue, 2-aminosuberic acid, or diamino propionic acid, and modified with a crosslinking agent.

**4.** The conjugate according to any one of claims 1 to 3, wherein the metal radionuclide is Ac-225, Y-90, Lu-177, or Zr-89.

**5.** The conjugate according to any one of claims 1 to 4, wherein the linker (L) comprises the formula (10a), the formula (10b), or the formula (10c):

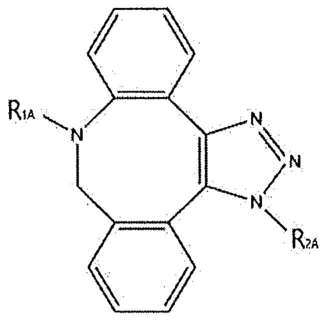

(10a)

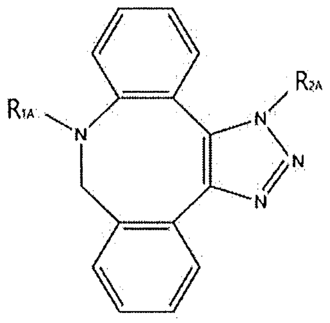

(10b)

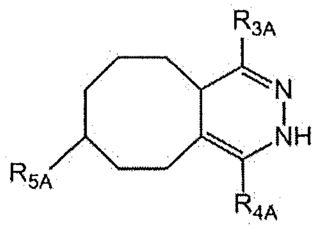

(10c)

.

in the formula (10a) and the formula (10b), $R_{1A}$ is a binding site with a chelating agent, and $R_{2A}$ is a binding site with the peptide. In the formula (10c), one of $R_{3A}$ and $R_{4A}$ is a hydrogen atom, a methyl group, a phenyl group or a pyridyl group, and the other is a binding site with the chelating agent, and $R_{5A}$ is a binding site with the peptide.

**6.** The conjugate according to claim 5, comprising a polyethylene glycol group between the linkage site with the peptide and the peptide.

**7.** The conjugate according to any one of claims 1 to 6, which is conjugated by a click reaction of an anti-HER2 antibody site-specifically modified with the peptide having an azide group introduced into the N-terminal, and a radioactive metal complex of DOTAGA-DBCO represented by the following formula:

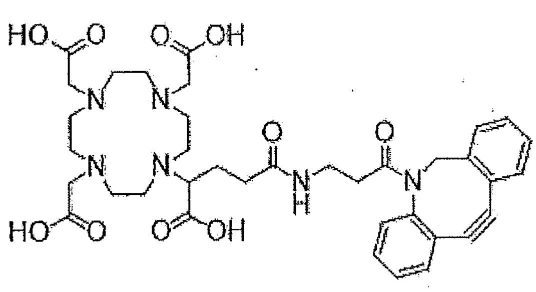

DOTAGA-DBCO

**8.** The conjugate according to any one of claims 1 to 7, wherein the anti-HER2 antibody is trastuzumab or pertuzumab.

**9.** A radiopharmaceutical comprising the conjugate according to any one of claims 1 to 8 as an active ingredient.

**10.** The radiopharmaceutical according to claim 9, which is used in a radionuclide therapy for cancer.

**11.** The radiopharmaceutical according to claim 9, which is used in cancer diagnosis.

**12.** The radiopharmaceutical according to claim 11, which is used in combination with the radionuclide therapy for cancer using a radiopharmaceutical according to claim 10.

**13.** A radiopharmaceutical containing a conjugate of a chelating agent chelated with a metal radionuclide and an anti-HER2 antibody as an active ingredient and satisfying the following condition (1) or (2), wherein
the linkage between the anti-HER2 antibody and the chelating agent does not contain a thiourea bond:

(1) the metal radionuclide is $^{177}$Lu or $^{90}$Y, and the conjugate has a radiochemical purity of not less than 90% when stored at room temperature for 7 days
(2) the metal radionuclide is $^{225}$Ac, and the conjugate has a radiochemical purity of not less than 90% when stored at room temperature for 14 days.

**14.** A radiopharmaceutical comprising a conjugate of a chelating agent chelated with a metal radionuclide and an anti-HER2 antibody as an active ingredient, wherein

the linkage between the anti-HER2 antibody and the chelating agent does not comprise a thiourea bond, and the conjugate has a radiochemical purity of not less than 90% at the time of expiry of a period that is a multiple of not less than 1 and not more than 5 of the half-life, based on the half-life of the metal radionuclide.

[Fig. 1]

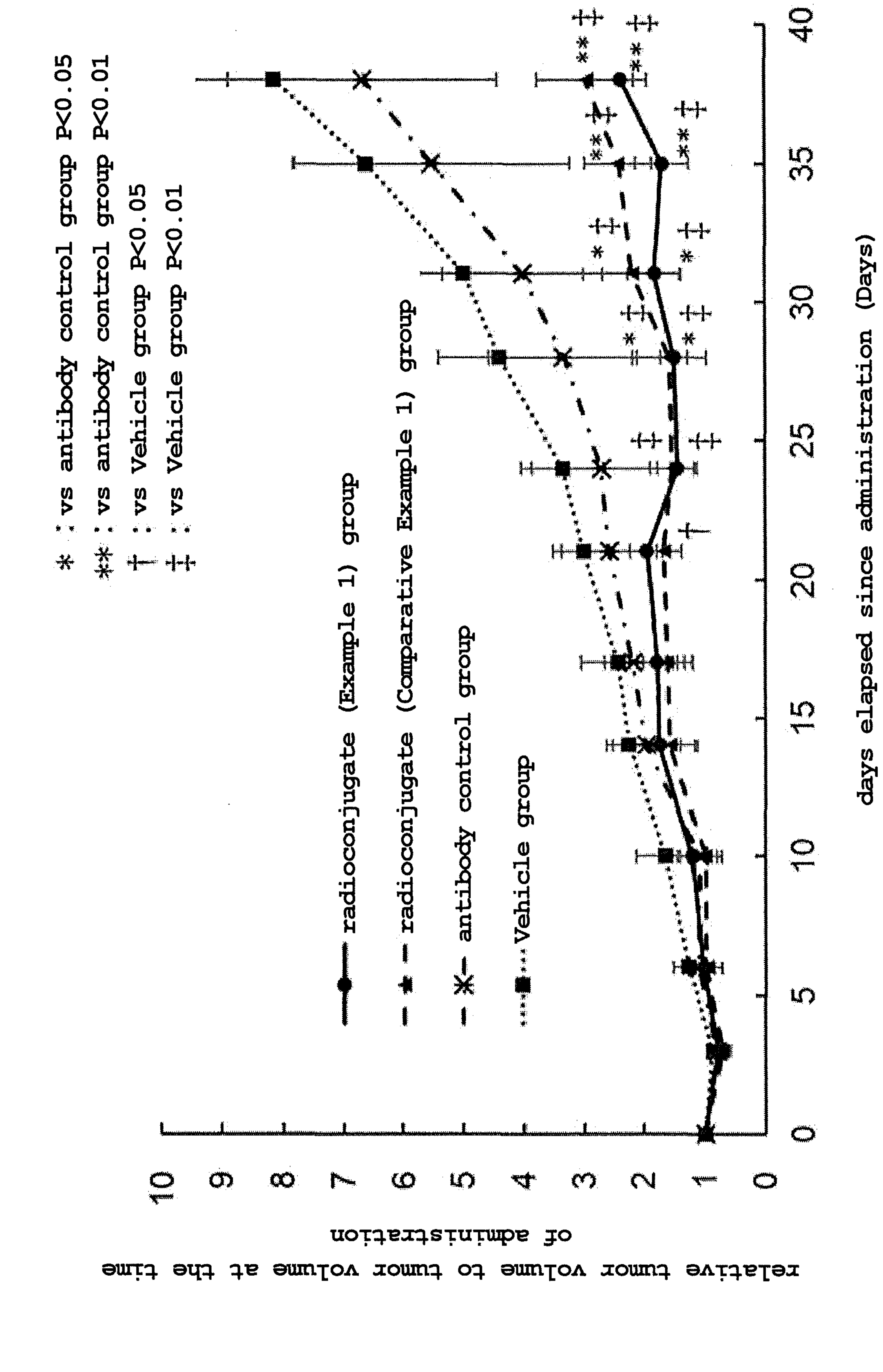
* : vs antibody control group P<0.05
** : vs antibody control group P<0.01
† : vs Vehicle group P<0.05
‡ : vs Vehicle group P<0.01
radioconjugate (Example 1) group
radioconjugate (Comparative Example 1) group
antibody control group
Vehicle group
relative tumor volume to tumor volume at the time of administration
days elapsed since administration (Days)
0
1
2
3
4
5
6
7
8
9
10
0
5
10
15
20
25
30
35
40

[Fig. 2]

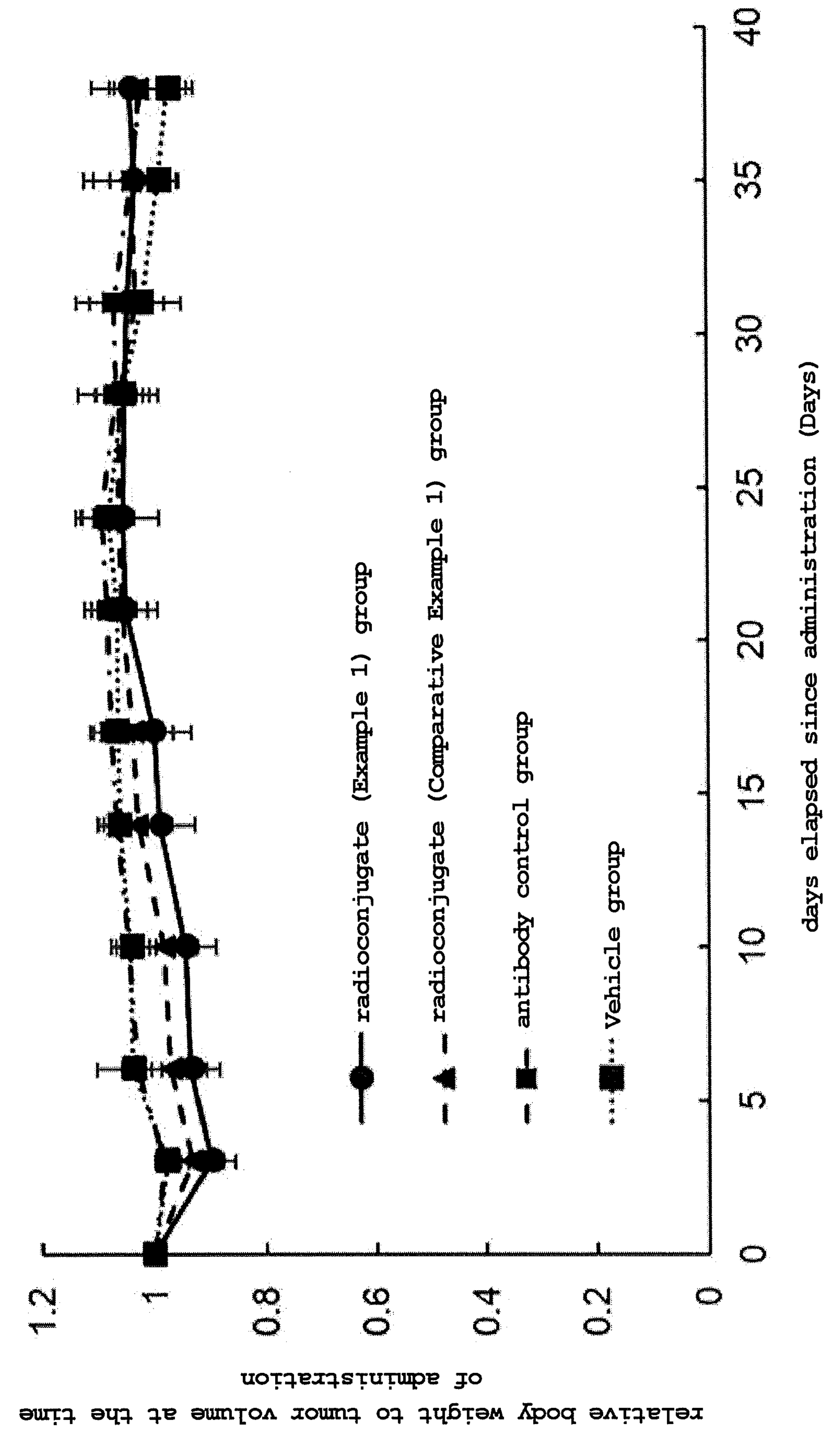
relative body weight to tumor volume at the time of administration
1.2
1
0.8
0.6
0.4
0.2
0
0
5
10
15
20
25
30
35
40
days elapsed since administration (Days)
radioconjugate (Example 1) group
radioconjugate (Comparative Example 1) group
antibody control group
Vehicle group

[Fig. 3]

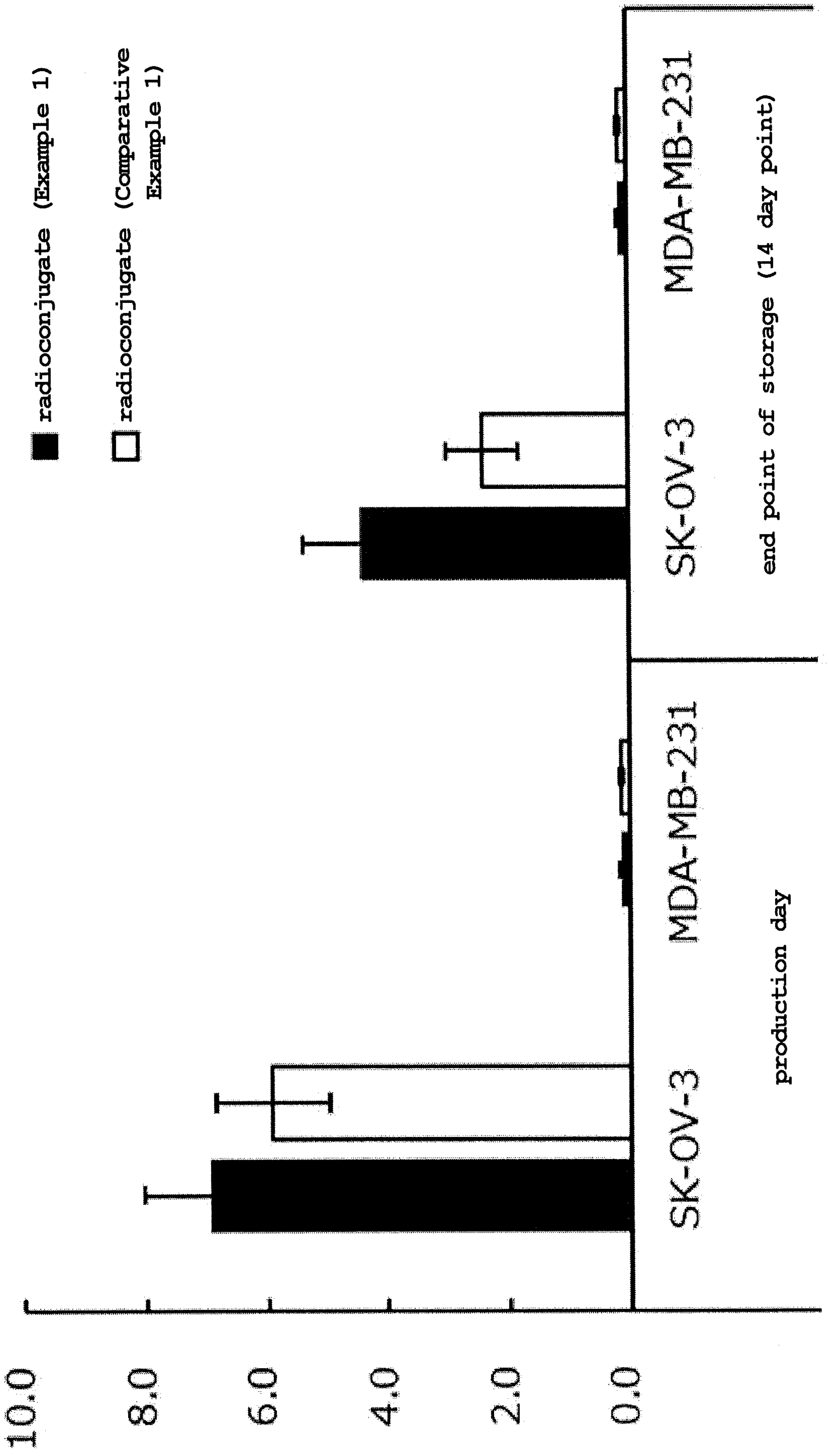
radioconjugate (Example 1)
radioconjugate (Comparative Example 1)
10.0
8.0
6.0
4.0
2.0
0.0
SK-OV-3
MDA-MB-231
production day
SK-OV-3
MDA-MB-231
end point of storage (14 day point)

[Fig. 4]

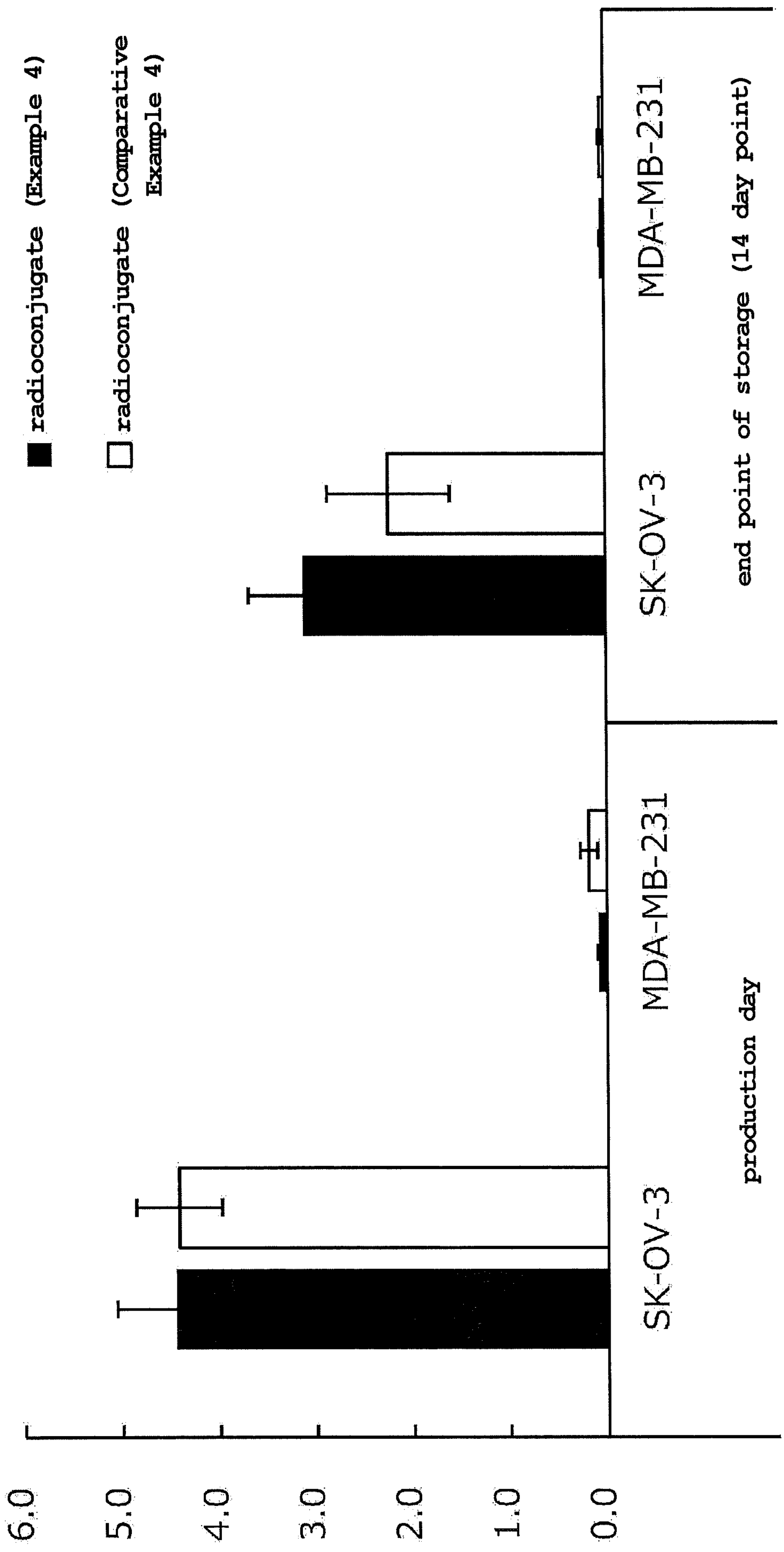
radioconjugate (Example 4)
radioconjugate (Comparative Example 4)
6.0
5.0
4.0
3.0
2.0
1.0
0.0
SK-OV-3
MDA-MB-231
SK-OV-3
MDA-MB-231
production day
end point of storage (14 day point)

[Fig. 5]

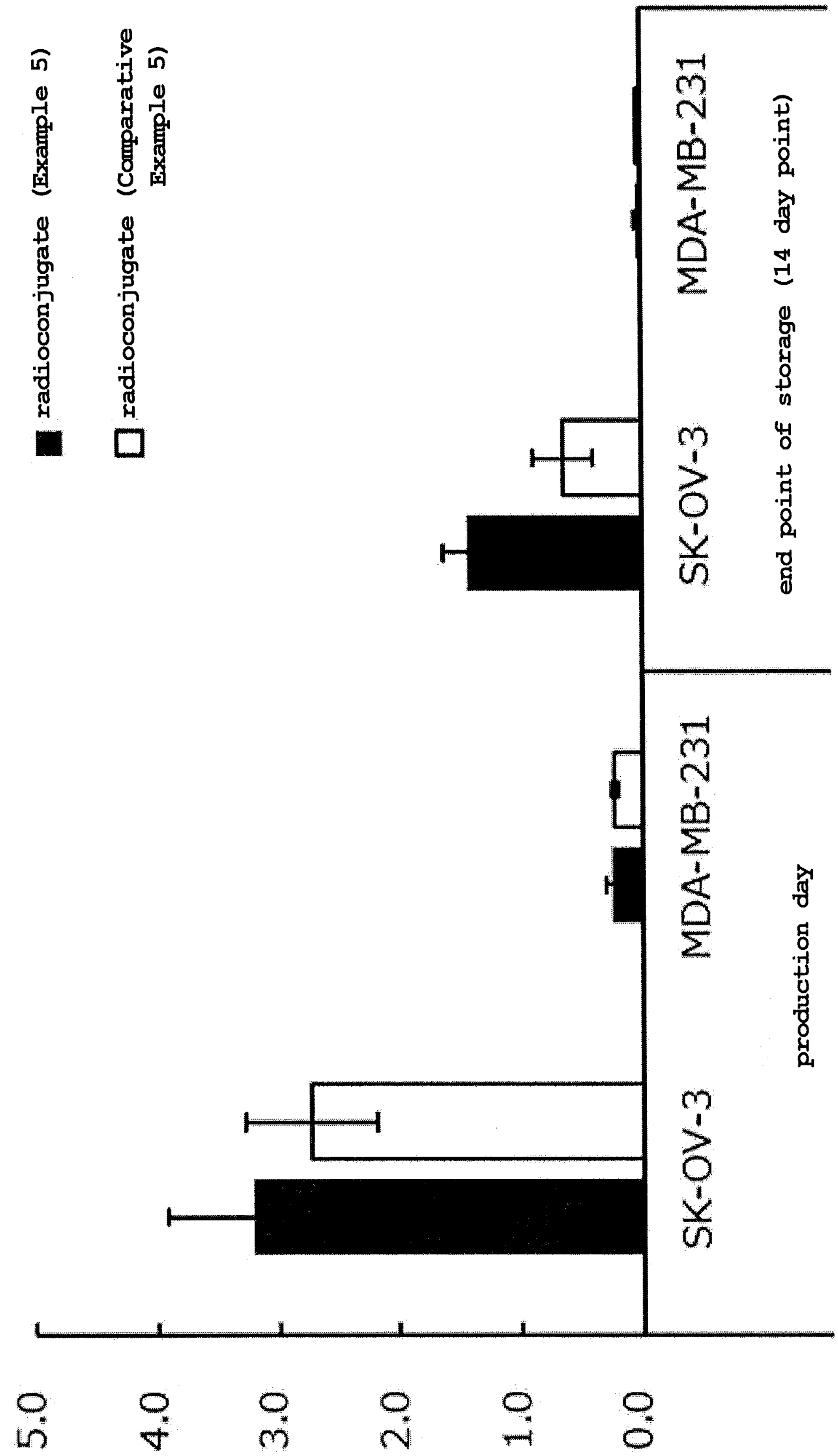
radioconjugate (Example 5)
radioconjugate (Comparative Example 5)
5.0
4.0
3.0
2.0
1.0
0.0
SK-OV-3
MDA-MB-231
SK-OV-3
MDA-MB-231
production day
end point of storage (14 day point)

[Fig. 6]

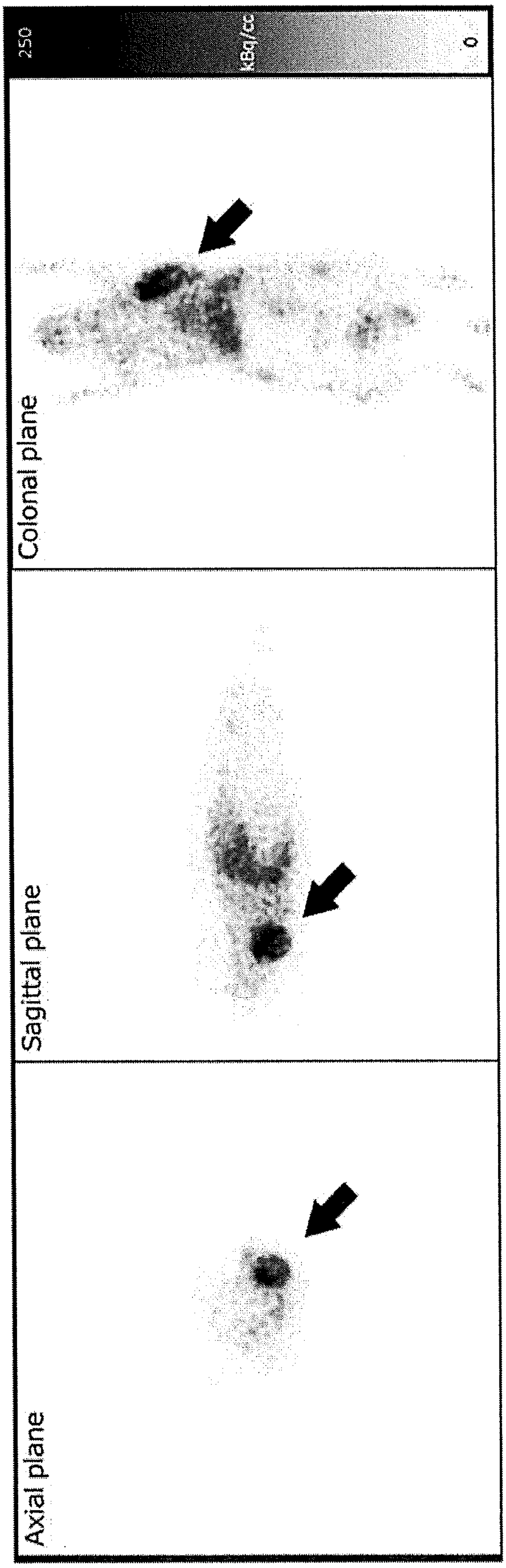

[Fig. 7]

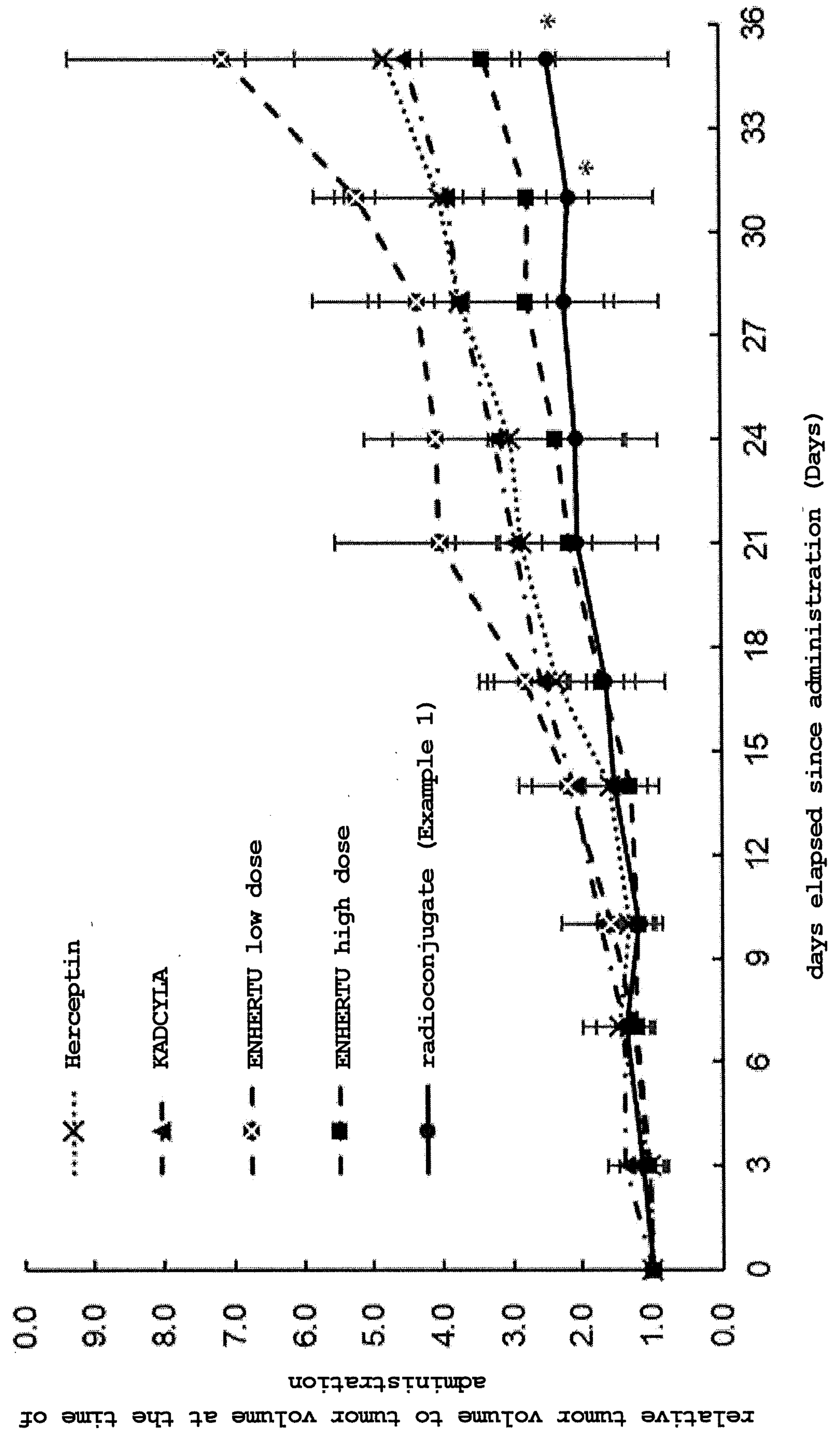
Herceptin
KADCYLA
ENHERTU low dose
ENHERTU high dose
radioconjugate (Example 1)
relative tumor volume to tumor volume at the time of administration
0.0
1.0
2.0
3.0
4.0
5.0
6.0
7.0
8.0
9.0
0.0
days elapsed since administration (Days)
0
3
6
9
12
15
18
21
24
27
30
33
36
*
*

[Fig. 8]

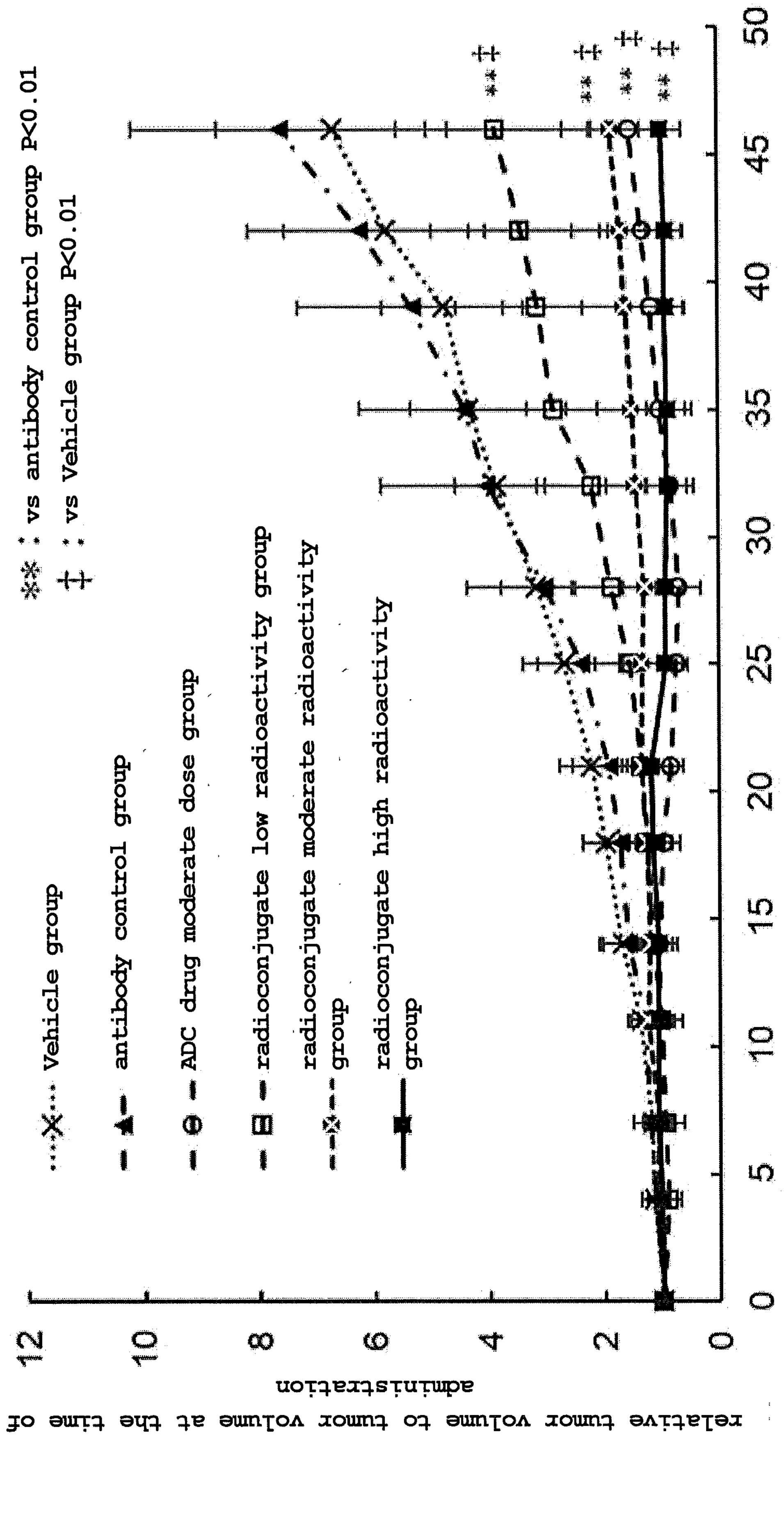
Vehicle group
antibody control group
ADC drug moderate dose group
radioconjugate low radioactivity group
radioconjugate moderate radioactivity group
radioconjugate high radioactivity group
※※ : vs antibody control group P<0.01
‡ : vs Vehicle group P<0.01
relative tumor volume to tumor volume at the time of administration
days elapsed since administration (Days)
0
2
4
6
8
10
12
0
5
10
15
20
25
30
35
40
45
50

**INTERNATIONAL SEARCH REPORT**

International application No.
**PCT/JP2021/038207**

**A. CLASSIFICATION OF SUBJECT MATTER**

***C07K 1/13***(2006.01)i; ***A61K 33/24***(2019.01)i; ***A61K 33/244***(2019.01)i; ***A61K 39/395***(2006.01)i; ***A61K 41/00***(2020.01)i; ***A61K 47/68***(2017.01)i; ***A61K 47/69***(2017.01)i; ***A61K 51/10***(2006.01)i; ***A61P 35/00***(2006.01)i; ***C07K 7/08***(2006.01)i; ***C07K 16/28***(2006.01)i; ***C07K 16/30***(2006.01)i; ***C12N 15/11***(2006.01)i

FI: C07K1/13; C07K16/30 ZNA; A61K41/00; A61K51/10 100; A61K47/69; A61K33/24; A61K51/10 200; A61K33/244; C12N15/11 Z; A61K39/395 L; C07K16/28 ZNA; A61P35/00; A61K39/395 T; A61K47/68; C07K7/08

According to International Patent Classification (IPC) or to both national classification and IPC

**B. FIELDS SEARCHED**

Minimum documentation searched (classification system followed by classification symbols)

C07K1/13; A61K33/24; A61K33/244; A61K39/395; A61K41/00; A61K47/68; A61K47/69; A61K51/10; A61P35/00; C07K7/08; C07K16/28; C07K16/30; C12N15/11

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2016/186206 A1 (KAGOSHIMA UNIVERSITY) 24 November 2016 (2016-11-24) examples 2, 9, paragraph [0053] | 1-14 |
| Y | WO 2019/125982 A1 (JANSSEN BIOTECH, INC.) 27 June 2019 (2019-06-27) pp. 4, 5, 12, fig. 1 | 1-14 |
| P, X | WO 2021/075546 A1 (NIHON MEDIPHYSICS CO., LTD. ) 22 April 2021 (2021-04-22) | 1-14 |
| P, A | WO 2021/075544 A1 (NIHON MEDIPHYSICS CO., LTD. ) 22 April 2021 (2021-04-22) | 1-14 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 December 2021** | **11 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/038207**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2021/038207**

| Patent document cited in search report | Publication date (day/month/year) | Patent family member(s) | Publication date (day/month/year) |
|---|---|---|---|
| WO 2016/186206 A1 | 24 November 2016 | US 2018/0141976 A1<br>examples 2, 9, paragraph [0256]<br>EP 3299383 A1<br>CA 2985985 A<br>SG 11201709573Y A<br>KR 10-2018-0002734 A<br>CN 107614514 A<br>JP 2020-203940 A | |
| WO 2019/125982 A1 | 27 June 2019 | US 2021/0017099 A1<br>EP 3727474 A1<br>CN 111491670 A<br>AU 2018388467 A<br>CA 3085465 A<br>JP 2021-506842 A | |
| WO 2021/075546 A1 | 22 April 2021 | (Family: none) | |
| WO 2021/075544 A1 | 22 April 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019125982 A
- WO 2016186206 A
- WO 2017217347 A
- JP H6508267 W
- WO 2018230257 A
- WO 2021080008 A
- JP 2020174840 A
- JP 2020215740 A
- JP 2021024688 A


### Non-patent literature cited in the description


- *Cancer Res.,* 15 August 2003, vol. 63 (16), 5084-90
- *Clin Cancer Res.,* 01 July 2004, vol. 10 (13), 4489-97
- **BERNHARD et al.** *DOTAGA-Anhydride: A Valuable Building Block for the Preparation of DOTA-Like Chelating Agents Chem. Eur. J.,* 2012, vol. 18, 7834-7841